# DEMANDE DE BREVET EUROPEEN

(11) **EP 2 184 271 A1**
(43) Date de publication de la demande: **12.05.2010**
(21) Numéro de dépôt: 09174130.6
(22) Date de dépôt: 27.10.2009
(51) Int. Cl.: C07C 229/26, A61K 8/44, A61Q 5/12

(54) **Utilisation d'au moins un composé dérivé de lysine pour le conditionnement des fibres kératiniques, composition cosmétique le contenant et procédé de conditionnement des fibres**

(30) Priorité: 27.10.2008 FR 0857287
(71) Demandeur: L'Oréal, 75008 Paris (FR)
(72) Inventeur: Ramos-Stanbury, Laure, 92330 Sceaux (FR); Peres Munoz, Lourdes, 08035 Barcelona (ES); Infante Martinez-Pardo, Maria, Rosa, 08008 Barcelona (ES)
(74) Mandataire: Dossmann, Gérard

(57) **Abrégé**

La présente invention concerne l'utilisation d'un ou plusieurs composés particuliers dérivés de lysine pour le conditionnement des fibres kératiniques, en particulier des fibres kératiniques humaines telles que les cheveux.

L'invention porte également sur une composition cosmétique de conditionnement des fibres kératiniques comprenant dans un milieu cosmétiquement acceptable, un ou plusieurs composés particuliers dérivés de lysine et un ou plusieurs adjuvants cosmétiques.

L'invention concerne plus particulièrement un procédé de conditionnement des fibres kératiniques en particulier humaines, mettant en oeuvre une composition cosmétique comprenant un ou plusieurs composés particuliers dérivés de lysine.

## Description

La présente invention concerne l'utilisation d'un ou plusieurs composés particuliers dérivés de lysine pour le conditionnement des fibres kératiniques, en particulier des fibres kératiniques humaines telles que les cheveux. L'invention porte également sur un procédé de conditionnement des fibres kératiniques mettant en oeuvre une composition comprenant ces composés.

L'invention a de même pour objet une composition cosmétique comprenant, dans un milieu cosmétiquement acceptable, un ou plusieurs composés particuliers dérivés de lysine ainsi que l'utilisation de cette composition pour le conditionnement des fibres.

Les cheveux sont généralement abîmés et fragilisés par l'action des agents atmosphériques extérieurs tels que la lumière et les intempéries, et par des traitements mécaniques ou chimiques tels que le brossage, le peignage, les teintures, les décolorations, les permanentes et/ou les défrisages.

Par exemple, dans le cas d'une permanente ou d'un défrisage, on a observé que la qualité des cheveux est souvent altérée. Les conséquences essentielles de cette altération sont une diminution de leurs propriétés cosmétiques, telles que leur brillance, ainsi qu'une dégradation de leurs propriétés mécaniques, en particulier une dégradation de leur résistance due à un gonflement des cheveux lors du rinçage entre l'étape d'application d'une composition contenant un agent réducteur (étape de réduction) et l'étape d'application d'une composition contenant un agent oxydant (étape d'oxydation). Cette dégradation peut également se traduire par une augmentation de la porosité des cheveux.

Les cheveux se retrouvent ainsi affaiblis et peuvent devenir cassants lors de traitements ultérieurs comme des brushings. Il en résulte également que de tels traitements rendent le plus souvent les cheveux difficiles à discipliner, en particulier ils sont difficiles à démêler ou à coiffer et les chevelures, même abondantes, conservent difficilement une coiffure de bon aspect en raison du fait que les cheveux manquent de vigueur et de nervosité.

Pour remédier à cela, il est maintenant usuel d'appliquer des produits de soin capillaires comprenant des agents de conditionnement, notamment des tensioactifs cationiques, afin de réparer ou limiter les effets néfastes ou indésirables induits par les différents traitements ou agressions que subissent, de manière plus ou moins répétée, les cheveux.

Ces produits de soin capillaires peuvent notamment se présenter sous la forme de gels, de crèmes, de lotions ou encore d'après-shampooings.

Cependant, ces produits de soin capillaire à base de tensioactifs cationiques ne confèrent généralement pas aux cheveux des propriétés cosmétiques entièrement satisfaisantes, notamment en termes de démêlage, de lissage, d'aptitude au peignage, de coiffage et de douceur.

Ainsi il existe un réel besoin de proposer des composés pour le conditionnement des cheveux afin de leur procurer des propriétés cosmétiques satisfaisantes, en particulier en termes de démêlage, de lissage, d'aptitude au peignage, de douceur et de toucher, et ceci tout en présentant une faible écotoxicité et en étant facilement biodégradables.

La demande de brevet internationale WO2006/056636 décrit des composés particuliers dérivés de lysine, tels que des composés du type ester de N,N-acyloxypropyl lysine de méthyle et ester de N,N-bis(N-acyloxypropyl)lysine de méthyle, leur procédé de synthèse ainsi que leur utilisation de manière générale dans le domaine alimentaire, pharmaceutique et cosmétique pour leur activité antimicrobienne et leur capacité à s'auto-agréger.

De manière surprenante et avantageuse, la demanderesse a maintenant découvert que l'utilisation de ces composés particuliers dérivés de lysine permettait de conditionner les cheveux, c'est-à-dire de conférer aux cheveux des propriétés cosmétiques satisfaisantes, en termes de démêlage, de lissage, d'aptitude au peignage, de douceur et de toucher, ceci tout en présentant une faible écotoxicité et en étant facilement biodégradables.

En particulier, il a été constaté que l'utilisation de ces composés particuliers dérivés de lysine permettait d'améliorer les propriétés cosmétiques qui sont conférées aux cheveux par rapport aux tensioactifs cationiques classiquement utilisés, notamment en ce qui concerne leur aptitude au démêlage, au peignage ainsi qu'en termes de douceur et de lissage.

De plus, par rapport aux tensioactifs classiquement utilisés, ces composés dérivés de lysine présentent une écotoxicité nettement réduite et sont plus facilement biodégradables.

L'invention a donc notamment pour objet l'utilisation pour le conditionnement des fibres kératiniques, en particulier des fibres kératiniques humaines telles que les cheveux, d'un ou plusieurs composés dérivés de lysine de formule (I) suivante : 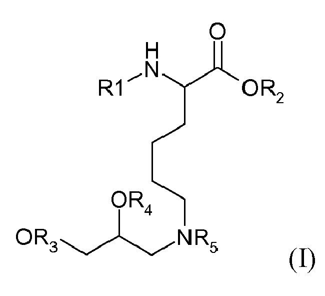 dans laquelle :
R₁ représente un atome d'hydrogène ou un groupement protecteur ;
R₂ représente un atome d'hydrogène ; un radical alkyle en C₁-C₆ linéaire ou un radical alkyle en C₃-C₆ ramifié ;
R₃ et R₄ représentent, indépendamment l'un de l'autre, un atome d'hydrogène ; un groupement acyle en C₂-C₂₅ linéaire, saturé ou insaturé, ou un groupement acyle en C₄-C₂₅ ramifié, saturé ou insaturé ;
R₅ représente un atome d'hydrogène ou un radical CH₂CH(OR₆)CH₂OR₇ ;
R₆ et R₇ représentent, indépendamment l'un de l'autre, un atome d'hydrogène ; un groupement acyle en C₃-C₂₅ linéaire, saturé ou insaturé, ou un groupement acyle en C₄-C₂₅ ramifié, saturé ou insaturé ;
R₁, R₂, R₃, R₄, R₅ et éventuellement R₆ et R₇, lorsqu'ils sont présents, ne peuvent pas représenter simultanément un atome d'hydrogène ;
ainsi que leurs sels et/ou leurs solvates.

Ces composés permettent notamment d'améliorer l'aptitude au démêlage des cheveux et l'apport de douceur par rapport aux tensioactifs cationiques qui sont classiquement utilisés dans des compositions cosmétiques.

Un autre objet de la présente invention consiste en un procédé de conditionnement des fibres kératiniques, en particulier des fibres kératiniques humaines telles que les cheveux, dans lequel on applique sur lesdites fibres une composition comprenant un ou plusieurs composés dérivés de lysine de formule (I) telle que définie ci-avant.

Plus particulièrement, la présente invention porte aussi sur une composition cosmétique pour le conditionnement des fibres kératiniques, qui comprend, dans un milieu cosmétiquement acceptable, un ou plusieurs composés de formule (I) telle que définie ci-avant et un ou plusieurs adjuvants cosmétiques.

Un autre objet de la présente demande porte sur des composés dérivés de lysine particuliers répondant à la formule (I) ainsi que sur une composition cosmétique pour le conditionnement des fibres kératiniques, qui comprend, dans un milieu cosmétiquement acceptable, de tels composés dérivés de lysine.

Mais d'autres caractéristiques, aspects, objets et avantages de la présente invention apparaîtront encore plus clairement à la lecture de la description et des exemples qui suivent.

Par groupement protecteur, on entend au sens de la présente invention, un groupement fonctionnel introduit dans la molécule pour protéger le groupement amine des réactions secondaires.

De préférence, le groupement protecteur est choisi parmi un groupement acyle en C₂-C₆, linéaire ou ramifié, de préférence un groupement acétyle ; un groupement alkyle en C₁-C₆, linéaire ou ramifié, de préférence un groupement méthyle ou éthyle ; un groupement carboxyalcoxy en C₁-C₆ ; un groupement alcoxy(C₁-C₆)carbonyle ; un groupement alkyle en C₁-C₆, linéaire ou ramifié, interrompu par un ou plusieurs atomes d'azote et/ou un groupement carboxy ; un groupement sulfonyle en C₂-C₆ ; un groupement aryle.

De préférence, le groupement protecteur est un groupement acyle en C₂-C₆, linéaire ou ramifié, de préférence un groupement acétyle.

De préférence, R₃ représente un groupement acyle en C₂-C₂₅ linéaire, saturé ou insaturé, ou un groupement acyle en C₄-C₂₅ ramifié, saturé ou insaturé.

De préférence, R₄ représente un atome d'hydrogène ou un groupement acyle en C₂-C₂₅ linéaire, saturé ou insaturé, ou un groupement acyle en C₄-C₂₅ ramifié, saturé ou insaturé.

Selon un premier mode de réalisation particulier, R₁ représente un atome d'hydrogène ; R₂ représente un radical alkyle en C₁-C₄ linéaire, de préférence un groupement méthyle ou éthyle, ou un radical alkyle en C₃-C₄ ramifié, de préférence un groupement tertiobutyle ; R₃ et R₄ représentent, indépendamment l'un de l'autre, un atome d'hydrogène ou un groupement acyle en C₁₂-C₂₂, de préférence en C₁₆-C₂₂, linéaire ou ramifié, saturé ou insaturé ; R₅ représente un atome d'hydrogène ou groupement CH₂CH(OR₆)CH₂OR₇ ; R₆ et R₇ représentent, indépendamment l'un de l'autre, un atome d'hydrogène ou un groupement acyle en C₁₂-C₂₂, de préférence en C₁₆-C₂₂, linéaire ou ramifié, saturé ou insaturé ; R₁, R₂, R₃, R₄, R₅ et éventuellement R₆ et R₇ ne peuvent pas représenter simultanément un atome d'hydrogène.

Selon un deuxième mode de réalisation plus particulier, R₁ représente un atome d'hydrogène ; R₂ représente un radical alkyle en C₃-C₄ ramifié, de préférence un groupement tertiobutyle ; R₃ représente un groupement acyle en C₁₂-C₂₂, de préférence en C₁₆-C₂₂, linéaire ou ramifié, saturé ou insaturé ; R₄ représente un atome d'hydrogène ; R₅ représente un groupement CH₂CH(OR₆)CH₂OR₇ , R₆ représente un atome d'hydrogène ou un groupement acyle en C₁₂-C₂₂, de préférence en C₁₆-C₂₂, linéaire ou ramifié, saturé ou insaturé ; R₇ représente un groupement acyle en C₁₂-C₂₂, de préférence en C₁₆-C₂₂, linéaire ou ramifié, saturé ou insaturé.

Selon une variante, R₁ représente un atome d'hydrogène ; R₂ représente un radical alkyle en C₁-C₄ linéaire, de préférence un groupement méthyle ou éthyle ; R₃ représente un groupement acyle en C₁₂-C₂₂, de préférence en C₁₆-C₂₂, linéaire ou ramifié, saturé ou insaturé ; R₄ représente un atome d'hydrogène ; R₅ représente un groupement CH₂CH(OR₆)CH₂OR₇ ; R₆ représente un atome d'hydrogène ou un groupement acyle en C₁₂-C₂₂, de préférence en C₁₆-C₂₂, linéaire ou ramifié, saturé ou insaturé ; R₇ représente un groupement acyle en C₁₂-C₂₂, de préférence en C₁₆-C₂₂, linéaire ou ramifié, saturé ou insaturé.

Ainsi, les composés dérivés de lysine de formule (I) peuvent être des esters qui sont mono-, di-, tri- ou tétra-substitués.

Conformément à un mode de réalisation particulièrement avantageux de l'invention, le ou les composés de formule (I) sont choisis dans le groupe formé:

| | |
|---|---|
| 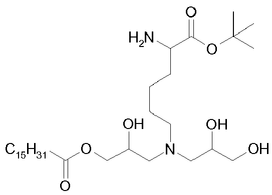 | 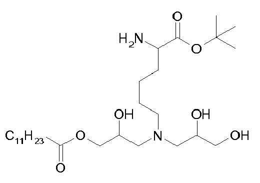 |
| Hexadecanoate de 3-[(5-amino-6-tert-butoxy-6-oxohexyl)(2,3-dihydroxypropyl)amino]-2-hydroxypropyl | dodécanoate de 3-[(5-amino-6-tert-butoxy-6-oxohexyl)(2,3-dihydroxypropyl)amino]-2-hydroxypropyl |
| 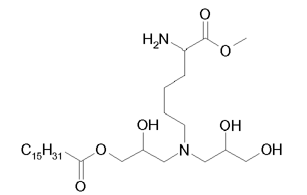 | 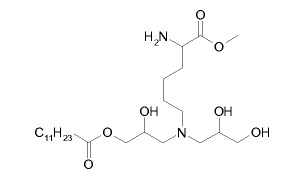 |
| hexadécanoate de 3-[(5-amino-6-methoxy-6-oxohexyl)(2,3-dihydroxypropyl)amino]-2-hydroxypropyl | dodécanoate de 3-[(5-amino-6-methoxy-6-oxohexyl)(2,3-dihydroxypropyl)amino]-2-hydroxypropyl |
| 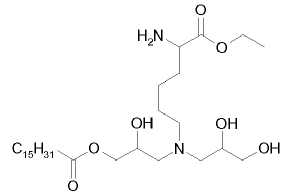 | 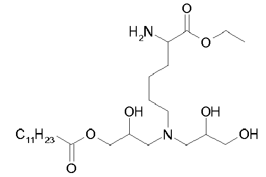 |
| hexadécanoate de 3-[(5-amino-6-ethoxy-6-oxohexyl)(2,3-dihydroxypropyl)amino]-2-hydroxypropyl | dodécanoate de 3-[(5-amino-6-ethoxy-6-oxohexyl)(2,3-dihydroxypropyl)amino]-2-hydroxypropyl |
| 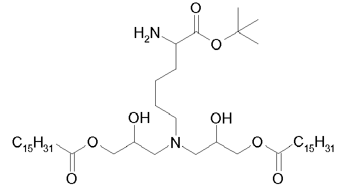 | 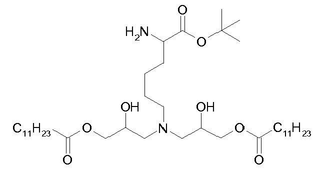 |
| dihexadécanoate de [(5-amino-6-tert-butoxy-6-oxohexyl)imino]bis-2-hydroxypropane-3,1-diyl | didodécanoate de [(5-amino-6-tert-butoxy-6-oxohexyl)imino]bis-2-hydroxypropane-3,1-diyl |
| 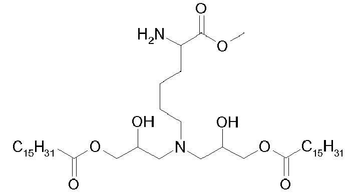 | 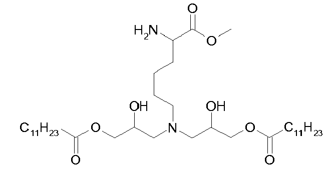 |
| dihexadécanoate de [(5-amino-6- méthoxy -6-oxohexyl)imino]bis-2-hydroxypropane-3,1-diyl | didodécanoate de [(5-amino-6- méthoxy -6-oxohexyl)imino]bis-2-hydroxypropane-3,1-diyl |
| 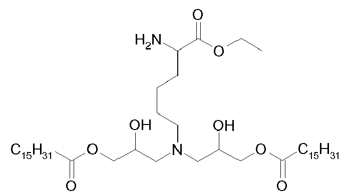 | 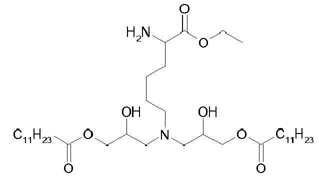 |
| dihexadécanoate de [(5-amino-6-éthoxy-6-oxohexyl)imino]bis-2-hydroxypropane-3,1-diyl | didodécanoate de [(5-amino-6-éthoxy -6-oxohexyl)imino]bis-2-hydroxypropane-3,1-diyl |
| 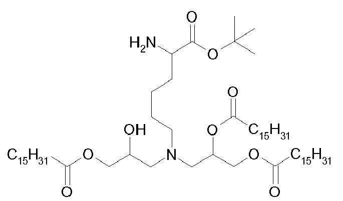 | 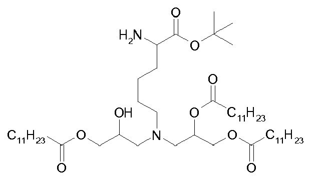 |
| palmitate de 3-{(5-amino-6-tert-butoxy-6-oxohexyl)[2,3-bis(palmitoyloxy)propyl]ammo}-2-hydroxypropyl | laurate de 3-{(5-amino-6-tert-butoxy-6-oxohexyl)[2,3-bis(dodecanoyloxy) propyl]amino}-2-hydroxypropyl |
| 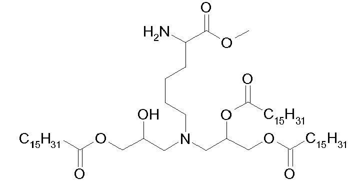 | 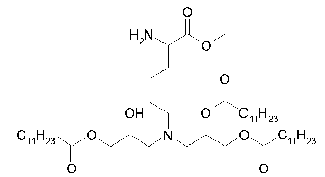 |
| palmitate de 3-{(5-amino-6-méthoxy-6-oxohexyl)[2,3-bis(palmitoyloxy)propyl]ammo}-2-hydroxypropyl | laurate de 3-{(5-amino-6-méthoxy-6-oxohexyl)[2,3-bis(dodecanoyloxy) propyl]amino}-2-hydroxypropyl |
| 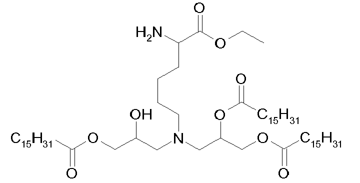 | 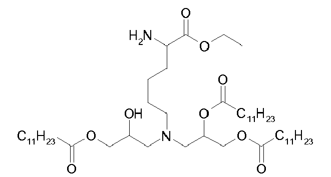 |
| palmitate de 3-{(5-amino-6-éthoxy-6-oxohexyl)[2,3-bis(palmitoyloxy)propyl]amino}-2-hydroxypropyl | laurate de 3-{(5-amino-6-éthoxy-6-oxohexyl)[2,3-bis(dodecanoyloxy) propyl]amino}-2-hydroxypropyl |
| 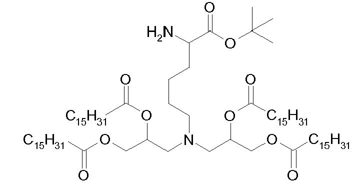 | 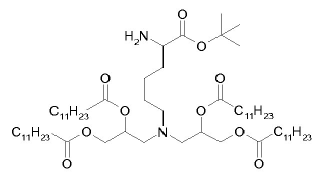 |
| tétrahexadécanoate de [(5-amino-6-tert-butoxy-6-oxohexyl)imino]dipropane-3,1,2-triyl | tétradodécanoate de [(5-amino-6-tert-butoxy-6-oxohexyl)imino]dipropane-3,1,2-triyl |
| 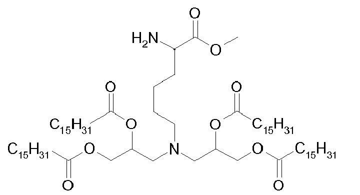 | 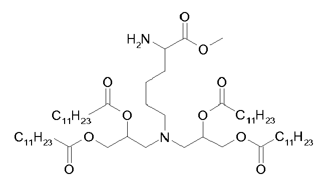 |
| tétrahexadécanoate de [(5-amino-6-méthoxy-6-oxohexyl)imino]dipropane-3,1,2-triyl | tétradodécanoate de [(5-amino-6-méthoxy-6-oxohexyl)imino]dipropane-3,1,2-triyl |
| 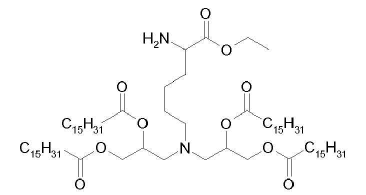 | 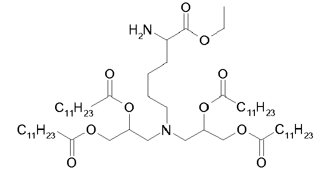 |
| tétrahexadécanoate de [(5-amino-6-éthoxy-6-oxohexyl)imino]dipropane-3,1,2-triyl | tétradodécanoate de [(5-amino-6-éthoxy-6-oxohexyl)imino]dipropane-3,1,2-triyl |
| 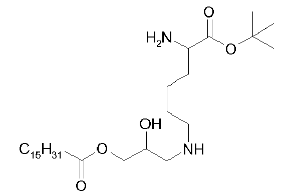 | 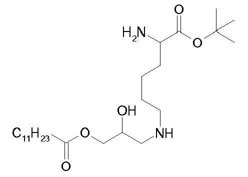 |
| palmitate de 3-[(5-amino-6-tert-butoxy-6-oxohexyl)amino]-2-hydroxypropyl | laurate de 3-[(5-amino-6-tert-butoxy-6-oxohexyl)amino]-2-hydroxypropyl |
| 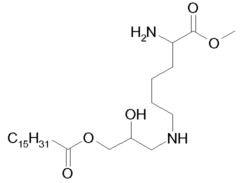 | 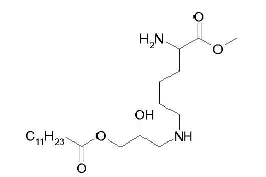 |
| palmitate de 3-[(5-amino-6-méthoxy-6-oxohexyl)amino]-2-hydroxypropyl | laurate de 3-[(5-amino-6- méthoxy-6-oxohexyl)amino]-2-hydroxypropyl |
| 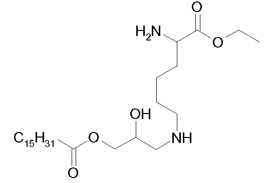 | 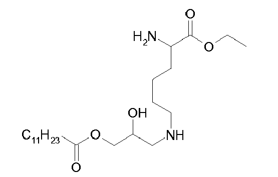 |
| palmitate de 3-[(5-amino-6-éthoxy-6-oxohexyl)amino]-2-hydroxypropyl | laurate de 3-[(5-amino-6- éthoxy-6-oxohexyl)amino]-2-hydroxypropyl |
| 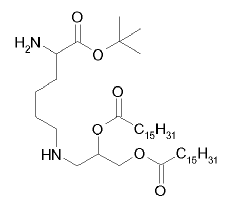 | 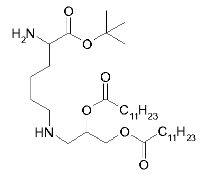 |
| dihexadécanoate de 3-[(5-amino-6-tert-butoxy-6-oxohexyl)amino]propane-1,2-diyl | didodécanoate de 3-[(5-amino-6-tert-butoxy-6-oxohexyl)amino]propane-1,2-diyl |
| 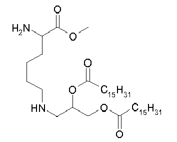 | 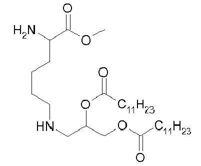 |
| dihexadécanoate 3-[(5-amino-6-méthoxy-6-oxohexyl)amino]propane-1,2-diyl | didodécanoate de 3-[(5-amino-6- méthoxy -6-oxohexyl)amino]propane-1,2-diyl |
| 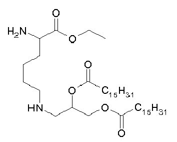 | 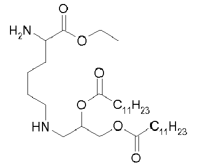 |
| dihexadécanoate de 3-[(5-amino-6-éthoxy-6-oxohexyl)amino]propane-1,2-diyl | didodécanoate de 3-[(5-amino-6- éthoxy -6-oxohexyl)amino]propane-1,2-diyl |
| 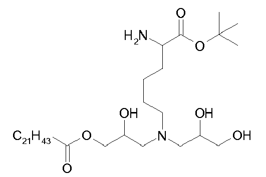 | |
| docosanoate de 3-[(5-amino-6-tert-butoxy-6-oxohexyl)(2,3-dihydroxypropyl)amino]-2-hydroxypropyl | |
| 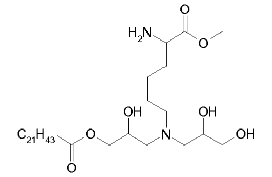 | |
| docosanoate de 3-[(5-amino-6-methoxy-6-oxohexyl)(2,3-dihydroxypropyl)amino]-2-hydroxypropyl | |
| 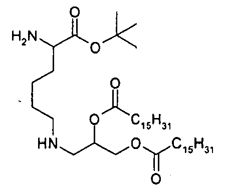 | 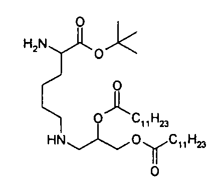 |
| dihexadécanoate de 3-[(5-amino-6-tert-butoxy-6-oxohexyl)amino]propane-1,2-diyl | didodécanoate de 3-[(5-amino-6-tert-butoxy-6-oxohèxyl)amino]propane-1,2-diyl |
| 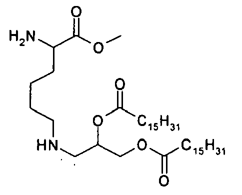 | 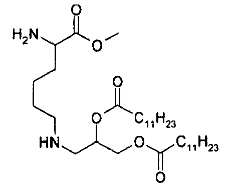 |
| dihexadécanoate 3-[(5-amino-6-méthoxy-6-oxohexyl)amino]propane-1,2-diyl | didodécanoate de 3-[(5-amino-6- méthoxy -6-oxohexyl)amino]propane-1,2-diyl |
| 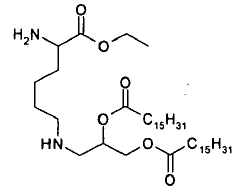 | 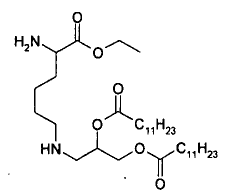 |
| dihexadécanoate de 3-[(5-amino-6-éthoxy-6-oxohexyl)amino]propane-1,2-diyl | didodécanoate de 3-[(5-amino-6- éthoxy -6-oxohexyl)amino]propane-1,2-diyl |
| 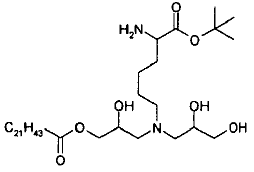 | |
| docosanoate de 3-[(5-amino-6-tert-butoxy-6-oxohexyl)(2,3-dihydroxypropyl)amino]-2-hydroxypropyl | |
| 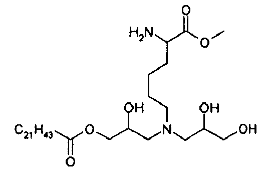 | |
| docosanoate de 3-[(5-amino-6-methoxy-6-oxohexyl)(2,3-dihydroxypropyl)amino]-2-hydroxypropyl | |
| 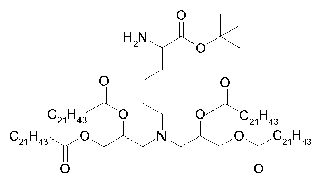 | 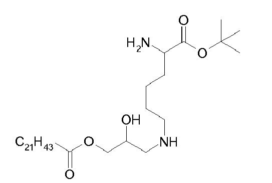 |
| Tétradocosanoate de [(5-amino-6-tert-butoxy-6-oxohexyl)imino]dipropane-3,1,2-triyl | docosanoate de 3-[(5-amino-6-tert-butoxy-6-oxohexyl)amino]-2-hydroxypropyl |
| 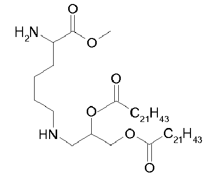 | 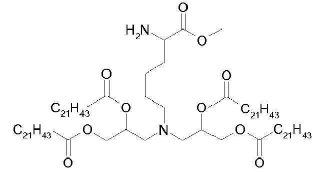 |
| didocosanoate de 3-[(5-amino-6-méthoxy -6-oxohexyl)amino]propane-1,2-diyl | Tétradocosanoate de [(5-amino-6-méthoxy-6-oxohexyl)imino]dipropane-3,1,2-triyl |
| 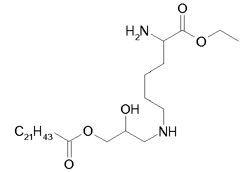 | 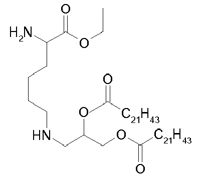 |
| docosanoate de 3-[(5-amino-6-éthoxy -6-oxohexyl)amino]-2-hydroxypropyl | didocosanoate de 3-[(5-amino-6-éthoxyl -6-oxohexyl)amino]propane-1,2-diyl |
| 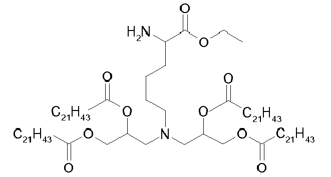 | |
| Tétradocosanoate de [(5-amino-6-éthyl-6-oxohexyl)imino]dipropane-3,1,2-triyl | |

De préférence, la présente invention concerne l'utilisation d'un mélange de plusieurs composés dérivés de lysine de formule (I), notamment d'esters mono-, di, tri et tétra substitués.

Par sel, on entend au sens de la présente invention, les sels organiques ou inorganiques d'un composé particulier dérivé de lysine de formule (I).

Parmi les sels inorganiques, on peut citer les halogénohydrates (chlorhydrates par exemple), les carbonates, les hydrogénocarbonates, les sulfates, les hydrogénophosphates, les phosphates.

Les sels organiques utilisables sont par exemple, les sels d'acides organiques comme les citrates, les lactates, les glycolates, les gluconates, les acétates, les propionates, les fumarates, les oxalates et les tartrates.

Par solvate, on entend, au sens de la présente invention, un mélange stoechiométrique dudit composé dérivé de lysine de formule (I) avec une ou plusieurs molécules d'eau ou de solvant organique.

Comme solvates possibles des composés dérivés de lysine de formule (I), on peut citer les hydrates, les alcoolates et les hydroalcoolates.

La synthèse de ces composés est connue et est par exemple décrite dans la demande de brevet internationale WO2006/056636.

La présente invention concerne également un procédé de conditionnement, des fibres kératiniques, en particulier des fibres kératiniques humaines telles que les cheveux, qui consiste à appliquer une composition cosmétique comprenant un ou plusieurs composés particuliers dérivés de lysine de formule (I) sur les fibres sèches ou humides, à rincer ou non après un éventuel temps de pose ou après un éventuel séchage.

De préférence, l'application de la composition cosmétique comprenant un ou plusieurs composés particuliers dérivés de lysine de formule (I) n'est pas suivie d'un rinçage.

L'invention porte plus particulièrement sur une composition de conditionnement des fibres kératiniques, en particulier des fibres kératiniques humaines telles que les cheveux, comprenant, dans un milieu cosmétiquement acceptable, un ou plusieurs composés particuliers dérivés de lysine de formule (I) telle que définie ci-avant et un ou plusieurs adjuvants cosmétiques.

Le ou les composés particuliers dérivés de lysine de formule (I) peuvent être présents dans une teneur en poids allant de 0,01 à 50% en poids, de préférence dans une teneur allant de 0,1 à 30 % en poids, notamment entre 0,5 et 25% en poids, voire entre 1 et 20% en poids, encore mieux entre 1,5 et 10% en poids, par rapport au poids total de la composition.

Le ou les adjuvants cosmétiques contenus dans la composition cosmétique sont choisis parmi les agents tensioactifs différents des composés de formule (I), les polymères, les silicones, les céramides et pseudo-céramides, les vitamines et pro-vitamines, les filtres solaires hydrosulbles et liposolubles, les composés solides tels que les pigments, les agents nacrants ou opacifiants, les agents séquestrants, les agents plastifiants, les agents solubilisants, les agents acidifiants, des agents alcalinisants, les agents neutralisants, les agents épaississants minéraux et organiques, les agents anti-mousse, les parfums, les agents alcalinisants, les agents anti-corrosion, les corps gras tels que les huiles végétales, animales, minérales et synthétiques, les agents réducteurs, les agents oxydants, les esters, les agents émollients, les agents anti-oxydants, les hydroxyacides, les solvants, les agents de pénétration, les tampons, les agents dispersants, les agents conservateurs et les agents de modification du pH.

Le ou les adjuvants cosmétiques est ou sont plus particulièrement choisis parmi les tensioactifs anioniques, cationiques, amphotères et non ioniques, les polymères cationiques, anioniques, amphotères ou zwittérioniques, non ioniques et les silicones.

Selon un mode de réalisation préférentiel, le ou les adjuvants cosmétiques est ou sont des tensioactifs choisis parmi les tensioactifs anioniques, cationiques, amphotères et non ioniques.

Les tensioactifs anioniques pouvant être utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (A) et (B) :

Rₐ-CONHCH₂CH₂-N(R_{b})(R_{c})(CH₂COO⁻) (A)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(B') (B)

dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Dans une variante préférée, la composition selon l'invention comprend un ou plusieurs tensioactifs anioniques et un ou plusieurs tensioactifs amphotères ou zwittérioniques.

A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Selon un autre mode réalisation préférentiel, le ou les adjuvants cosmétiques est ou sont choisis parmi les polymères tels que les polymères cationiques, amphotères (tels que zwitterioniques), et non ioniques, de préférence les polymères cationiques et des associations de ceux-ci. On entend par « polymère » tel qu'utilisé ici les homopolymères et les copolymères, les copolymères étant issus de plus d'un type de monomère, par exemple de deux, trois, quatre types de monomères différents ou plus.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (II), (III), (IV) ou (V) suivantes: 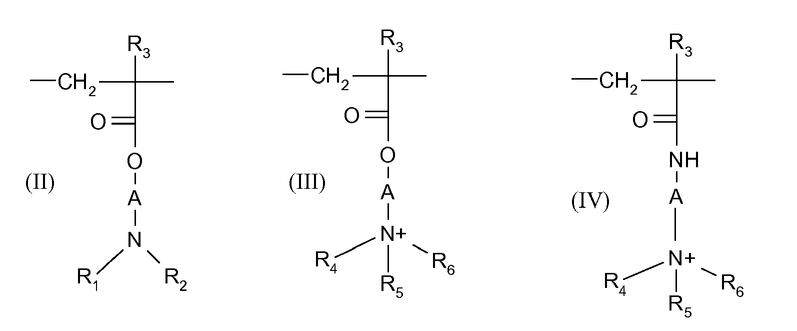 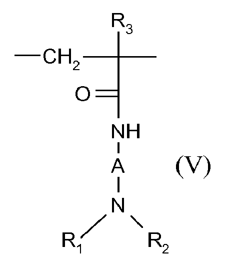 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un halogénure de diméthyle;
   - les copolymères d'acrylamide et de chlorure de méthacryloyloxyéthyltriméthylammonium décrits par exemple dans la demande de brevet EP-A-080976;
   - le copolymère d'acrylamide et de méthosulfate de méthacryloyloxyéthyltriméthylammonium;
   - les copolymères vinylpyrrolidone / acrylate ou méthacrylate de dialkylaminoalkyle quaternisés ou non. Ces polymères sont décrits en détail dans les brevets français 2.077.143 et 2.393.573;
   - les terpolymères méthacrylate de diméthyl amino éthyle/vinylcaprolactame/ vinylpyrrolidone;
   - les copolymère vinylpyrrolidone / méthacrylamidopropyl diméthylamine;
   - les copolymères vinylpyrrolidone / méthacrylamide de diméthylaminopropyle quaternisés
   - les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société CIBA.
(2) Les polysaccharides cationiques et en particulier ceux choisis parmi :
   (a) les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
   (b) les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylmidopropyl triméthylammonium, de diméthyl-diallylammonium.
   (c) les polygalactomannanes cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(4) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(5) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine.
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(7) Les cyclopolymères d'alkyl diallyl amine ou de dialkyl diallyl ammonium tels que les homopolymères ou copolymères comportant comme constituant principal de la chaîne des motifs répondant aux formules (VI) ou (VII) : 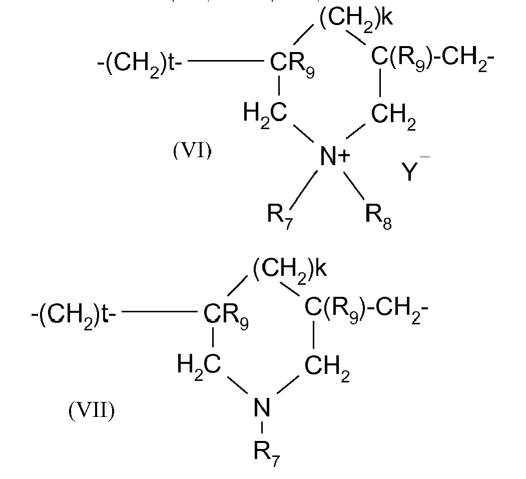 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
(8) Le polymère de diammonium quaternaire contenant des motifs récurrents répondant à la formule : 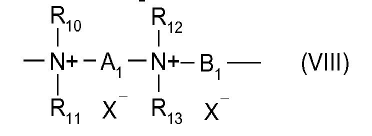 formule (VIII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (IX) suivante: 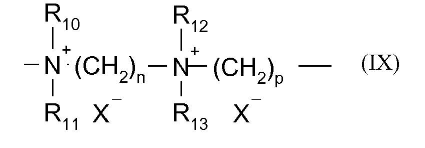 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(9) Les polymères de poly(ammonium quaternaire) constitués de motifs récurrents de formule (X) : 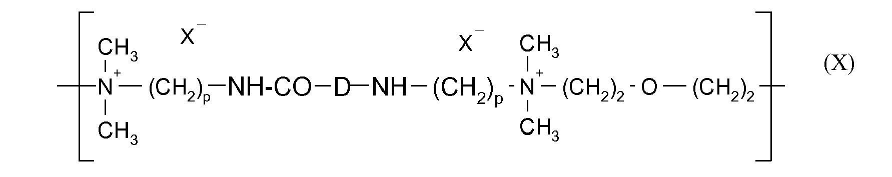 dans laquelle p désigne un nombre entier variant de 1 à 6 environ, D peut être nul ou peut représenter un groupement -(CH₂)ᵣ - CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
(10) Les polymères quaternaires de vinylpyrrolidone et de vinylimidazole.
(11) Les polyamines comme le produit référencé sous le nom de " POLYETHYLENEGLYCOL (15) TALLOW POLYAMINE " dans le dictionnaire CTFA.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques utilisables selon la présente invention, on préfère notamment :
- les dérivés d'éther de cellulose quaternaires tels que les produits commercialisés sous la dénomination « JR 400 » par la société Rhodia Chimie, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société Rhodia Chimie, les homopolymères et les copolymères éventuellement réticulés de sel de (méth)acryloyloxyéthyltriméthylammonium, vendus par la société CIBA en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.

Selon un mode de réalisation particulièrement préféré, les compositions selon l'invention comprennent un ou plusieurs polymères cationiques choisis parmi les homopolymères ou les copolymères du chlorure de diméthyldiallylammonium.

Les polymères amphotères utilisables dans les compositions de la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl triméthyl ammonium.
   Le composé vinylique peut être également un sel de dialkyldiallylammonium tel que le chlorure de diméthyldiallylammonium.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
(3) Les polyaminoamides réticulés et alcoylés partiellement ou totalement dérivant de polyaminoamides de formule générale :

   ⁅CO-R₁₉-CO-Z⁆ (XI)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 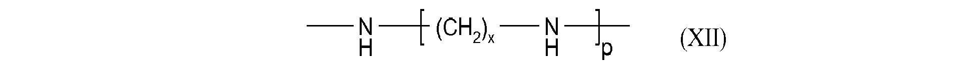 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XII) ci-dessus, dans lequel x=2 et p=1 et qui dérive de l'éthylènediamine, ou le radical dérivant de la pipérazine : 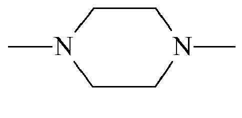
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 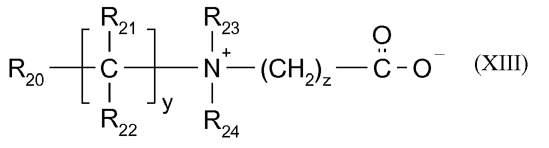 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane comportant des motifs monomères répondant aux formules (XIV), (XV), (XVI) suivantes : 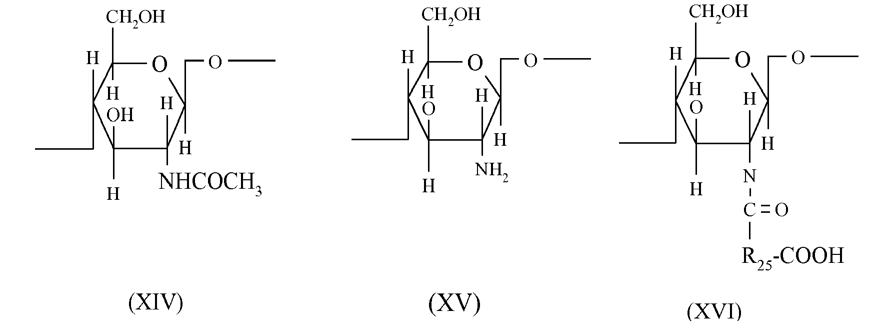 le motif (XIV) étant présent dans des proportions comprises entre 0 et 30%, le motif (XV) dans des proportions comprises entre 5 et 50% et le motif (XVI) dans des proportions comprises entre 30 et 90%, étant entendu que dans ce motif (XVI), R₂₅ représente un radical de formule : 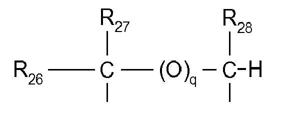 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane.
(7) Les polymères répondant à la formule générale (XVII) tels que ceux décrits par exemple dans le brevet français 1 400 366 et comprenant des unités : 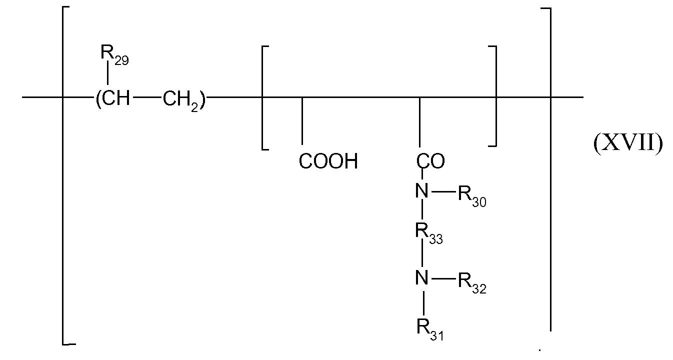 dans laquelle R₂₉ représente un atome d'hydrogene, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)_{2,} R₃₃ représentant un groupement -CH₂-CH₂- , -CH₂-CH₂-CH₂- , -CH₂-CH(CH₃)- , R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVIII)

      où D désigne un radical 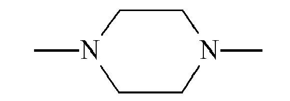 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XIX)

      où D désigne un radical 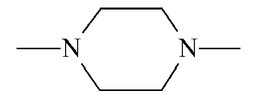 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi tous les polymères amphotères utilisables dans la composition selon la présente invention, on préfère notamment :
- le copolymère chlorure de diméthyldiallylammonium/acide acrylique (80/20) commercialisé sous la dénomination MERQUAT 280 DRY par la société CALGON (dénomination CTFA : POLYQUATERNIUM 22);
- le copolymère chlorure de diméthyldiallylammonium/acide acrylique (95/5) commercialisé sous la dénomination MERQUAT 295 DRY par la société CALGON (dénomination CTFA : POLYQUATERNIUM 22);
- le copolymère de chlorure de méthacrylamidopropyltrimonium, d'acide acrylique et d'acrylate d'éthyle, commercialisé sous la dénomination MERQUAT 2001 par la société CALGON (dénomination CTFA : POLYQUATERNIUM 47); et
- le terpolymère acrylamide/chlorure de diméthyldiallylammonium/acide acrylique, commercialisé sous la dénomination MERQUAT PLUS 3330 DRY par la société CALGON (dénomination CTFA : POLYQUATERNIUM 39).

Les polymères non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines vendues par la société Dow Chemical sous les noms PEOX 50 000, PEOX 200 000 et PEOX 500 000 ;
- les homopolymères d'acétate de vinyle tels que le produit vendu sous le nom Appretan EM par la société Hoechst ou le produit vendu sous le nom Rhodopas A 012 par la société Rhodia Chimie ;
- les copolymères d'acétate de vinyle et d'ester acrylique tels que le produit vendu sous le nom Rhodopas AD 310 par Rhodia Chimie;
- les copolymères d'acétate de vinyle et d'éthylène tels que le produit vendu sous le nom Appretan TV par la société Hoechst ;
- les copolymères d'acétate de vinyle et d'ester maléïque, par exemple de maléate de dibutyle tels que le produit vendu sous le nom Appretan MB Extra par la société Hoechst ;
- les copolymères de polyéthylène et d'anhydride maléïque ;
- les homopolymères d'acrylate d'alkyle et les homopolymères de méthacrylate d'alkyle tels que le produit vendu sous le nom Micropearl RQ 750 par la société Matsumoto ou le produit vendu sous le nom Luhydran A 848 S par la société BASF ;
- les copolymères d'esters acryliques tels que par exemple les copolymères de (méth)acrylates d'alkyle, tels que les produits vendus par la société Rohm & Haas sous les noms Primal AC-261 K et Eudragit NE 30 D, par la société BASF sous les noms Acronal 601, Luhydran LR 8833 ou 8845, et par la société Hoechst sous les noms Appretan N 9213 ou N 9212 ;
- les copolymères d'acrylonitrile et d'un monomère non ionique choisis par exemple parmi le butadiène et les (méth)acrylates d'alkyle ; on peut citer les produits vendus sous les noms Nipol LX 531 B par la société Nippon Zeon ou ceux vendus sous le nom CJ0601 B par la société Rohm & Haas ;
- les polyuréthanes tels que les produits vendus sous les noms Acrysol RM 1020 ou Acrysol RM 2020 par la société Rohm & Haas, et les produits Uraflex XP 401 UZ et Uraflex XP 402 UZ par la société DSM Resins ;
- les copolymères d'acrylate d'alkyle et d'uréthanne tels que le produit 8538-33 par la société National Starch ;
- les polyamides tels que le produit Estapor LO 11 vendu par la société Rhodia Chimie ;
- les gommes de guar non ioniques chimiquement modifiées ou non modifiées.

Les gommes de guar non ioniques non modifiées sont par exemple les produits vendus sous le nom Vidogum GH 175 par la société Unipectine et sous le nom Jaguar C par la société Meyhall.

Les gommes de guar non ioniques modifiées utilisables selon l'invention sont par exemple modifiées par des groupements hydroxyalkyle en C₁-C₆. On peut citer à titre d'exemples les groupements hydroxyméthyle, hydroxyéthyle, hydroxypropyle et hydroxybutyle.

Ces gommes de guar sont bien connues dans la technique antérieure et peuvent être préparées par exemple en faisant réagir des oxydes d'alcène correspondants tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les noms commerciaux Jaguar HP8, Jaguar HP60, Jaguar HP120, Jaguar DC 293, et Jaguar HP 105 par la société Meyhall et sous le nom Galactosol 4H4FD2 par la société Aqualon.

Les groupements alkyles des polymères non ioniques comprennent de 1 à 6 atomes de carbone, sauf mention contraire.

Il est également possible d'utiliser, comme polymères, des polyuréthanes fonctionnalisés ou non et siliconés ou non.

Des exemples de polyuréthanes utiles incluent ceux divulgués dans les brevets EP 0 751 162, EP 0 637 600, FR 2 743 297, EP 0 648 485, EP 0 656 021, WO 94/03510 et EP 0 619 111.

Dans un autre mode de mise en oeuvre, les polymères peuvent être utilisés sous forme solubilisée ou peuvent être sous forme de dispersions de particules solides ou liquides (latex ou pseudolatex).

Selon un autre mode de réalisation, le ou les adjuvants cosmétiques est ou sont choisis parmi les silicones.

Dans tout ce qui suit, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou par polycondensation de silanes convenablement fonctionnalisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles dont la partie alkyle est en C₁-C₁₀, les radicaux aryles et en particulier phényle.

Les silicones utilisées dans la composition selon l'invention peuvent être volatiles ou non, solubles ou insolubles dans la composition. Elles peuvent être en particulier des polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les organopolysiloxanes sont définis plus en détail dans l'ouvrage de Walter NOLL "Chemistry and Technology of Silicones" (1968) Academic Press. Ils peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 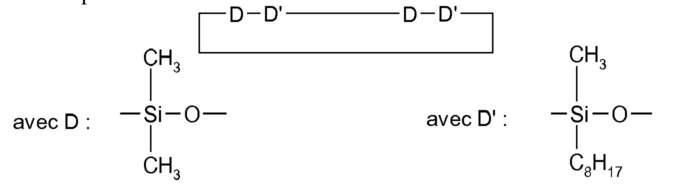 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Par silicone non volatile, on entend au sens de la présente invention, toute silicone dont le nombre d'atomes de silicium est supérieure à 7.

Parmi les silicones non volatiles, on peut notamment citer des polyalkylsiloxanes, des polyarylsiloxanes, des polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société Rhodia ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société Rhodia.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyldiphénylsiloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de RHODIA,
- les huiles des séries RHODORSIL 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

On peut plus particulièrement citer les produits suivants :
- les gommes de polydiméthylsiloxane,
- les gommes de polydiméthylsiloxanes/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane/diphénylsiloxane/méthylvinylsiloxane.

Peuvent également être employés des mélanges de silicones tels que :
- les mélanges formés à partir d'une gomme de polydiméthylsiloxane hydroxylée en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkylcarboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

De préférence, la silicone est une silicone aminée.

Par silicone aminé, on entend au sens de la présente invention, toute silicone comportant au moins une fonction amine primaire, secondaire, tertiaire ou un groupement ammonium quaternaire.

Les silicones aminées utilisées dans la composition sous forme de film selon la présente invention sont choisies parmi :
(a) les composés répondant à la formule (XX) suivante :

   (R¹)ₐ(T)₃₋ₐ-Si[OSi(T)₂]ₙ-[OSi(T)_{b}(R¹)_{2-b}]m-OSi(T)₃₋ₐ-(R¹)ₐ (XX)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -C_{q}H_{2q}L dans laquelle q est un nombre de 2 à 8 et L est un groupement aminé éventuellement quaternisé choisi parmi les groupements :

   -N(R2)-CH₂-CH₂-N(R²)₂ ;

   -N(R²)₂ ; -N⁺(R²)₃ Q- ;

   -N⁺(R²)(H)₂ Q- ;

   -N⁺(R²)₂HQ- ;

   -N(R²)-CH₂-CH₂-N⁺(R²)(H)₂ Q⁻,

   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.
   En particulier, les silicones aminées correspondant à la définition de la formule (XX) sont choisies parmi les composés correspondant à la formule suivante (XXI) : 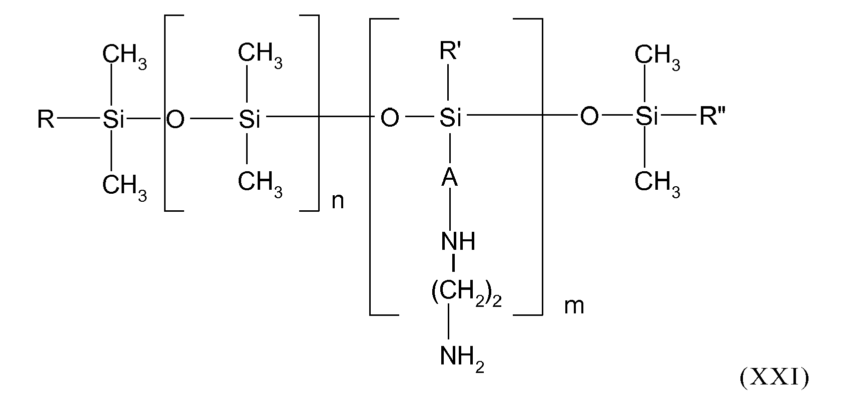 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.
   Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".
   Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.
   Dans cette catégorie de composés, on peut citer entre autres, le produit Belsil®ADM 652, commercialisée par Wacker.
   Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.
   Plus particulièrement, on peut citer le produit FluidWR® 1300, commercialisé par Wacker.
   Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).
   Un produit correspondant à la définition de la formule (XX) est en particulier le polymère dénommé dans le dictionnaire CTFA "triméthylsilylamodiméthicone", répondant à la formule (XXII) suivante: 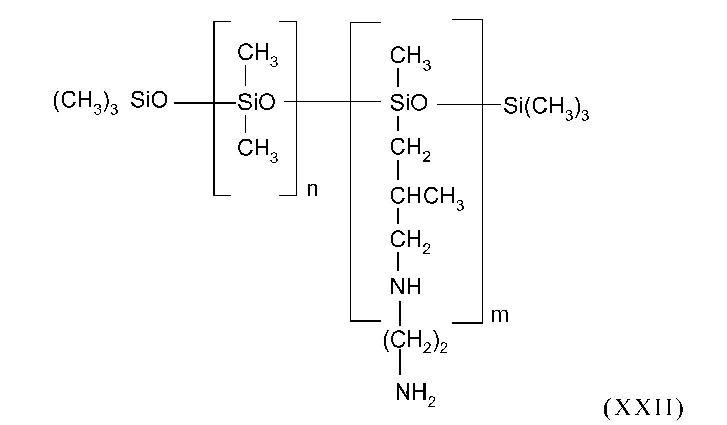 dans laquelle n et m ont les significations données ci-dessus conformément à la formule (XXII).
   De tels composés sont décrits par exemple dans EP 95238; un composé de formule (XX) est par exemple vendu sous la dénomination Q2-8220 par la société OSI.
(b) les composés répondant à la formule (XXIV) suivante : 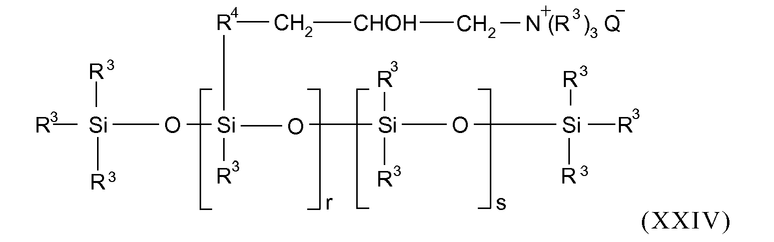 dans laquelle,
   R³ représente un radical hydrocarboné monovalent en C₁-C₁₈, et en particulier un radical alkyle en C₁-C₁₈, ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈ , par exemple en C₁-C₈,
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.
   De tels composés sont décrits plus particulièrement dans le brevet US 4185087.
   Un composé entrant dans cette classe est celui vendu par la Société Union Carbide sous la dénomination "Ucar Silicone ALE 56".
c) les silicones ammonium quaternaire de formule (XXV) : 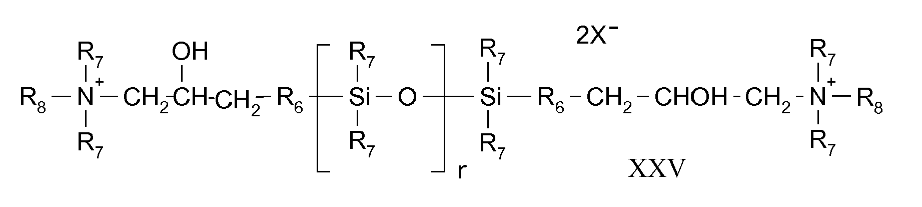
dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈ , un radical -R₆-NHCOR₇ ,
X- est un anion tel qu'un ion halogénure, notamment chlorure ou un sel d'acide organique (acétate ...);
r représente une valeur statistique moyenne de 2 à 200 et en particulier de 5 à 100 ;
Ces silicones sont par exemple décrites dans la demande EP-A-0530974.

Selon l'invention, on peut également utiliser des polymères du type silicone greffée comprenant une portion polysiloxane et une portion comprenant une chaîne organique non siliconée, l'une des deux portions constituant la chaîne principale du polymère et l'autre étant greffée sur la chaîne principale. Ces polymères sont divulgués, par exemple, dans les documents EP A 0 412 704, EP-A-0 412 707, EP-A-0 640 105, WO 95/00578, EP-A-0 582 152 et WO 93/23009 et les brevets U.S. n° 4 693 935, 4 728 571 et 4 972 037. Ces polymères sont par exemple anioniques ou non ioniques.

De tels polymères sont par exemple des copolymères susceptibles d'être obtenus par polymérisation radicalaire à partir du mélange de monomères comprenant :
a) environ 50 % à environ 90 % en poids d'acrylate de tert-butyle ;
b) 0 % à environ 40 % en poids d'acide acrylique ;
c) environ 5 % à environ 40 % en poids de macromère siliconé de formule : 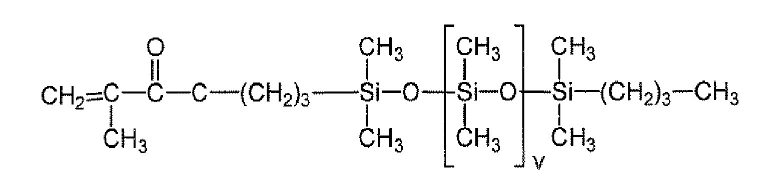
dans laquelle v est un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'une chaîne de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'une chaîne de raccordement de type thiopropylène, des motifs polymères du type poly(méth)acrylate d'isobutyle.

De préférence, le ou les adjuvants cosmétiques sont choisis parmi les tensioactifs cationiques autres que les composés de formule (I), les polymères cationiques et les silicones aminées.

Le ou les adjuvants cosmétiques est ou sont présents dans une teneur allant de 0,01 à 50% en poids, notamment 0,05 à 40% en poids, voire 0,1 à 30% en poids, par rapport au poids total de la composition.

Par milieu cosmétiquement acceptable, on entend un milieu compatible avec les matières kératiniques et en particulier les cheveux.

Le milieu cosmétiquement acceptable peut être un milieu alcoolique, aqueux ou hydroalcoolique. Ainsi le milieu peut notamment être constitué uniquement par de l'eau ou de l'alcool ou par un mélange d'eau et d'un ou de plusieurs solvants cosmétiquement acceptables tels que les alcools inférieurs en C₁-C₄, les polyols, les monoéthers de polyols et leurs mélanges. De préférence, l'alcool est l'éthanol.

De préférence, la composition cosmétique est une composition rincée. On entend par « composition rincée » toute composition qui est formulée pour être rincée immédiatement ou après un temps de pose inférieur à 30 minutes, de préférence inférieur à 10 minutes, après application sur les cheveux.

La composition rincée peut se présenter sous toutes les formes classiques de compositions cosmétiques rincées y compris, mais sans s'y limiter, les shampoings, après-shampoings, lotions de rinçage des cheveux, compositions de permanente, compositions de teinture des cheveux, produits à utiliser avant ou après un traitement de teinture des cheveux, produits à utiliser avant ou après un traitement de permanente, compositions de défrisage, produits à utiliser avant ou après un traitement de défrisage, et des associations de ceux-ci. Un shampoing présente un effet nettoyant sur les cheveux et peut également présenter un effet conditionneur. Un après-shampooing présente un effet conditionneur sur les cheveux sans effet nettoyant marqué.

De préférence, la composition selon l'invention est une composition rincée, en particulier un shampooing ou un après-shampooing.

La présente invention a également pour objet un ou plusieurs composés dérivés de lysine particuliers répondant à la formule (I) choisis parmi :

| | |
|---|---|
| 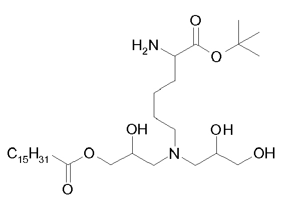 | 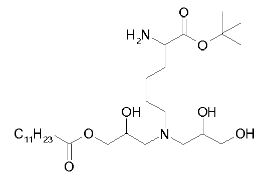 |
| Hexadecanoate de 3-[(5-amino-6-tert-butoxy-6-oxohexyl)(2,3-dihydroxypropyl)amino]-2-hydroxypropyl | dodécanoate de 3-[(5-amino-6-tert-butoxy-6-oxohexyl)(2,3-dihydroxypropyl)amino]-2-hydroxypropyl |
| 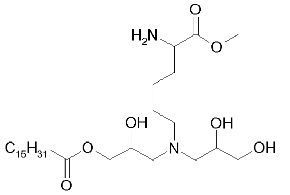 | 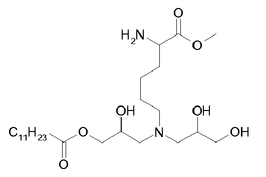 |
| hexadécanoate de 3-[(5-amino-6-methoxy-6-oxohexyl)(2,3-dihydroxypropyl)amino]-2-hydroxypropyl | dodécanoate de 3-[(5-amino-6-methoxy-6-oxohexyl)(2,3-dihydroxypropyl)amino]-2-hydroxypropyl |
| 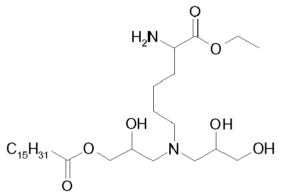 | 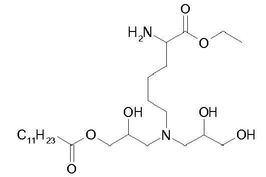 |
| hexadécanoate de 3-[(5-amino-6-ethoxy-6-oxohexyl)(2,3-dihydroxypropyl)amino]-2-hydroxypropyl | dodécanoate de 3-[(5-amino-6-ethoxy-6-oxohexyl)(2,3-dihydroxypropyl)amino]-2-hydroxypropyl |
| 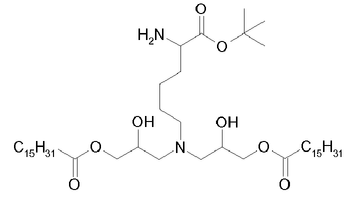 | 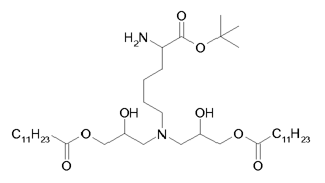 |
| dihexadécanoate de [(5-amino-6-tert-butoxy-6-oxohexyl)imino]bis-2-hydroxypropane-3,1-diyl | didodécanoate de [(5-amino-6-tert-butoxy-6-oxohexyl)imino]bis-2-hydroxypropane-3,-diyl |
| 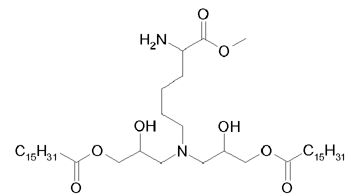 | |
| dihexadécanoate de [(5-amino-6- méthoxy -6-oxohexyl)imino]bis-2-hydroxypropane-3,1-diyl | |
| 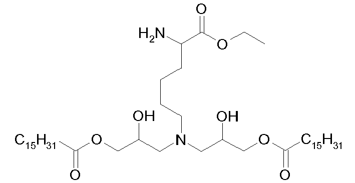 | 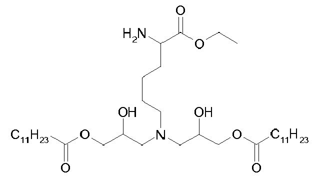 |
| dihexadécanoate de [(5-amino-6-éthoxy-6-oxohexyl)imino]bis-2-hydroxypropane-3,1-diyl | didodécanoate de [(5-amino-6-éthoxy -6-oxohexyl)imino]bis-2-hydroxypropane-3,1-diyl |
| 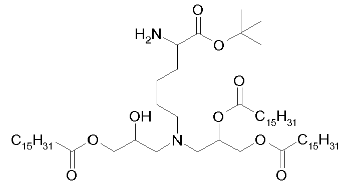 | 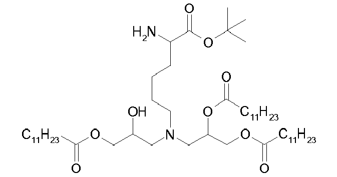 |
| palmitate de 3-{(5-amino-6-tert-butoxy-6-oxohexyl)[2,3-bis(palmitoyloxy)propyl]amino}-2-hydroxypropyl | laurate de 3-{(5-amino-6-tert-butoxy-6-oxohexyl)[2,3-bis(dodecanoyloxy) propyl]amino}-2-hydroxypropyl |
| 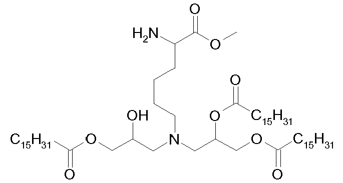 | 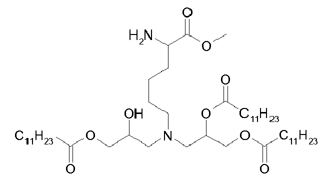 |
| palmitate de 3-{(5-amino-6-méthoxy-6-oxohexyl)[2,3-bis(palmitoyloxy)propyl]amino}-2-hydroxypropyl | laurate de 3-{(5-amino-6-méthoxy-6-oxohexyl)[2,3-bis(dodecanoyloxy) propyl]amino}-2-hydroxypropyl |
| 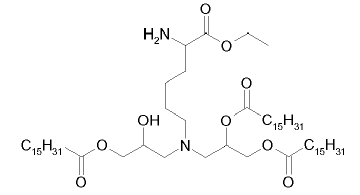 | 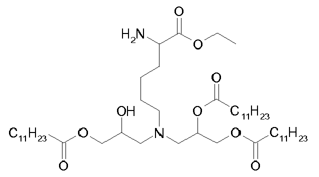 |
| palmitate de 3-{(5-amino-6-éthoxy-6-oxohexyl)[2,3-bis(palmitoyloxy)propyl]amino}-2-hydroxypropyl | laurate de 3-{(5-amino-6-éthoxy-6-oxohexyl)[2,3-bis(dodecanoyloxy) propyl]amino}-2-hydroxypropyl |
| 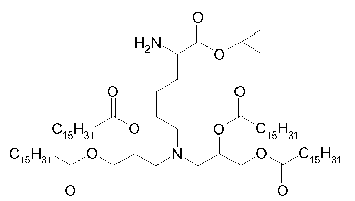 | 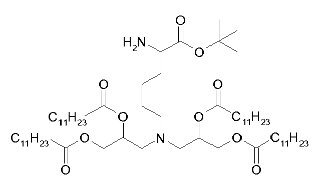 |
| tétrahexadécanoate de [(5-amino-6-tert-butoxy- 6-oxohexyl)imino]dipropane-3,1,2-triyl | tétradodécanoate de [(5-amino-6-tert-butoxy-6-oxohexyl)imino]dipropane-3,1,2-triyl |
| 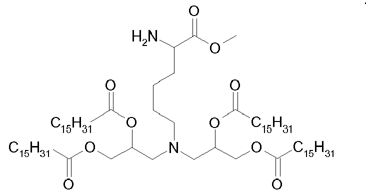 | 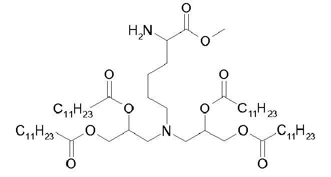 |
| tétrahexadécanoate de [(5-amino-6-méthoxy-6-oxohexyl)imino]dipropane-3,1,2-triyl | tétradodécanoate de [(5-amino-6-méthoxy-6-oxohexyl)imino]dipropane-3,1,2-triyl |
| 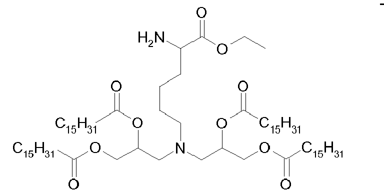 | 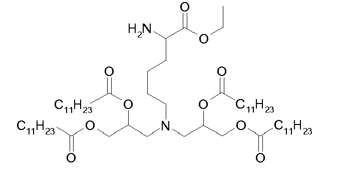 |
| tétrahexadécanoate de [(5-amino-6-éthoxy-6-oxohexyl)imino]dipropane-3,1,2-triyl | tétradodécanoate de [(5-amino-6-éthoxy-6-oxohexyl)imino]dipropane-3,1,2-triyl |
| 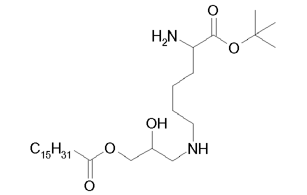 | 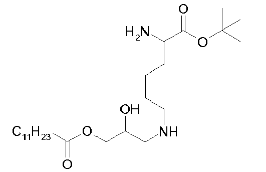 |
| palmitate de 3-[(5-amino-6-tert-butoxy-6-oxohexyl)amino]-2-hydroxypropyl | laurate de 3-[(5-amino-6-tert-butoxy-6-oxohexyl)amino]-2-hydroxypropyl |
| 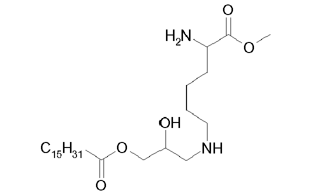 | 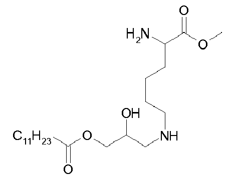 |
| palmitate de 3-[(5-amino-6-méthoxy-6-oxohexyl)amino]-2-hydroxypropyl | laurate de 3-[(5-amino-6- méthoxy-6-oxohexyl)amino]-2-hydroxypropyl |
| 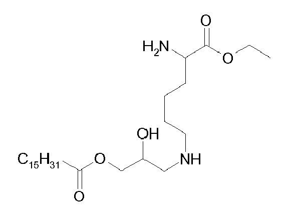 | 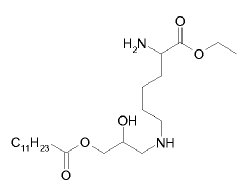 |
| palmitate de 3-[(5-amino-6-éthoxy-6-oxohexyl)amino]-2-hydroxypropyl | laurate de 3-[(5-amino-6- éthoxy-6-oxohexyl)amino]-2-hydroxypropyl |
| 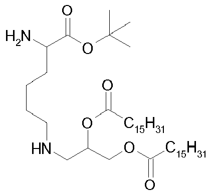 | 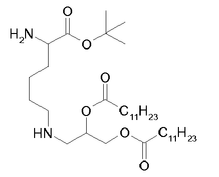 |
| dihexadécanoate de 3-[(5-amino-6-tert-butoxy-6-oxohexyl)amino]propane-1,2-diyl | didodécanoate de 3-[(5-amino-6-tert-butoxy-6-oxohexyl)amino]propane-1,2-diyl |
| 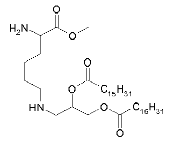 | 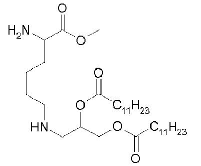 |
| dihexadécanoate 3-[(5-amino-6-méthoxy-6-oxohexyl)amino]propane-1,2-diyl | didodécanoate de 3-[(5-amino-6- méthoxy -6-oxohexyl)amino]propane-1,2-diyl |
| 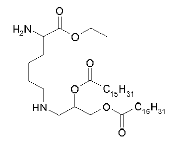 | 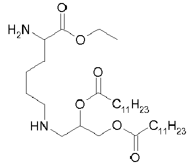 |
| dihexadécanoate de 3-[(5-amino-6-éthoxy-6-oxohexyl)amino]propane-1,2-diyl | didodécanoate de 3-[(5-amino-6- éthoxy -6-oxohexyl)amino]propane-1,2-diyl |
| 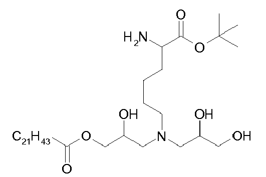 | |
| docosanoate de 3-[(5-amino-6-tert-butoxy-6-oxohexyl)(2,3-dihydroxypropyl)amino]-2-hydroxypropyl | |
| 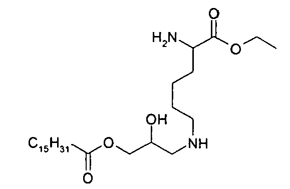 | 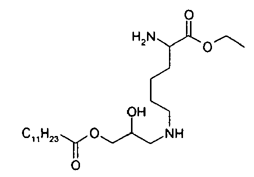 |
| palmitate de 3-[(5-amino-6-éthoxy-6-oxohexyl)amino]-2-hydroxypropyl | laurate de 3-[(5-amino-6- éthoxy-6-oxohexyl)amino]-2-hydroxypropyl |
| 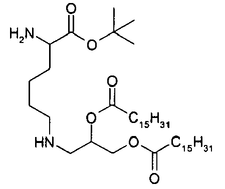 | 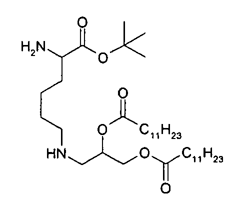 |
| dihexadécanoate de 3-[(5-amino-6-tert-butoxy-6-oxohexyl)amino]propane-1,2-diyl | didodécanoate de 3-[(5-amino-6-tert-butoxy-6-oxohexyl)amino]propane-1,2-diyl |
| 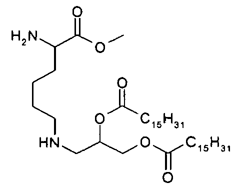 | 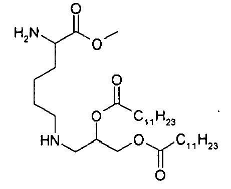 |
| dihexadécanoate 3-[(5-amino-6-méthoxy-6-oxohexyl)amino]propane-1,2-diyl | didodécanoate de 3-[(5-amino-6- méthoxy -6-oxohexyl)amino]propane-1,2-diyl |
| 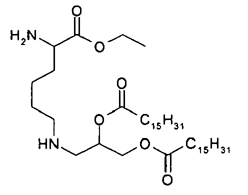 | 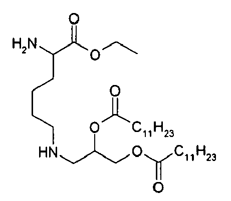 |
| dihexadécanoate de 3-[(5-amino-6-éthoxy-6-oxohexyl)amino]propane-1,2-diyl | didodécanoate de 3-[(5-amino-6- éthoxy -6-oxohexyl)amino]propane-1,2-diy) |
| 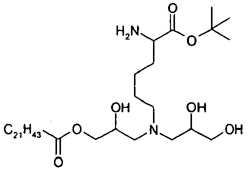 | |
| docosanoate de 3-[(5-amino-6-tert-butoxy-6-oxohexyl)(2,3-dihydroxypropyl)amino]-2-hydroxypropyl | |
| 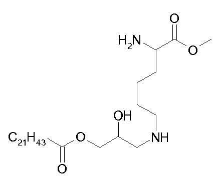 | 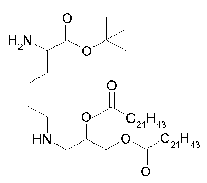 |
| docosanoate de 3-[(5-amino-6-méthoxy -6-oxohexyl)amino]-2-hydroxypropyl | didocosanoate de 3-[(5-amino-6-tert-butoxy-6-oxohexyl)amino]propane-1,2-diyl |
| 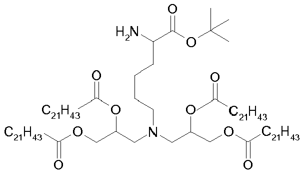 | 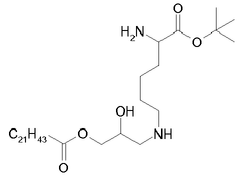 |
| Tétradocosanoate de [(5-amino-6-tert-butoxy-6-oxohexyl)imino]dipropane-3,1,2-triyl | docosanoate de 3-[(5-amino-6-tert-butoxy-6-oxohexyl)amino]-2-hydroxypropyl |
| 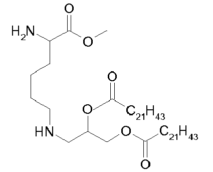 | 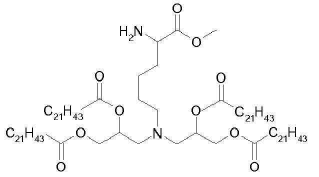 |
| didocosanoate de 3-[(5-amino-6-méthoxy -6-oxohexyl)amino]propane-1,2-diyl | Tétradocosanoate de [(5-amino-6-méthoxy-6-oxohexyl)imino]dipropane-3,1,2-triyl |
| 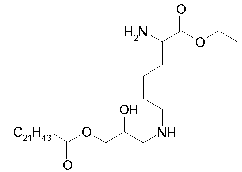 | 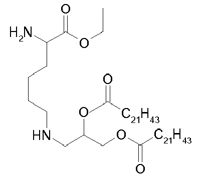 |
| docosanoate de 3-[(5-amino-6-éthoxy -6-oxohexyl)amino]-2-hydroxypropyl | didocosanoate de 3-[(5-amino-6-éthoxyl -6-oxohexyl)amino]propane-1,2-diyl |
| 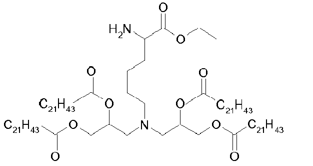 | |
| Tétradocosanoate de [(5-amino-6-éthyl-6-oxohexyl)imino]dipropane-3,1,2-triyl | |

De préférence, la présente invention concerne un mélange de plusieurs composés dérivés de lysine de formule (I), notamment des esters mono-, di-, tri- et tétra- substitués.

De préférence, le mélange de composés dérivés de lysine comprend de 0,5 à 15% en poids de mono-esters, de 25 à 99,5% en poids de di-esters, de 0,5 à 70% en poids de triesters et de 0,5 à 70% en poids de tétra-esters, par rapport au poids total du mélange.

Selon un mode de réalisation particulier, le mélange de composés dérivés de lysine comprend 6 % en poids d'hexadécanoate de 3-[(5-amino-6-methoxy-6-oxohexyl)(2,3-dihydroxypropyl)amino]-2-hydroxypropyl, 55% en poids de dihexadecanoate de [(5-amino-6-méthoxy-6-oxohexyl)imino]bis-2-hydroxypropane-3,1-diyl et 36% en poids d'un mélange de palmitate de 3-{(5-amino-6-méthoxy-6-oxohexyl)[2,3-bis(palmitoyloxy)propyl]amino}-2-hydroxypropyl et de tétrahexadécanoate de [(5-amino-6-méthoxy-6-oxohexyl)imino]dipropane-3,1,2-triyl.

De plus, la présente invention concerne une composition cosmétique pour le conditionnement des fibres kératiniques, en particulier des fibres kératiniques humaines telles que les cheveux, comprenant un ou plusieurs composés dérivés de lysine particuliers tels que définis ci-avant.

De plus, la présente invention a également pour objet l'utilisation en cosmétique d'un ou plusieurs composés dérivés de lysine tels que définis ci-avant.

Les exemples suivants sont donnés à titre illustratif et non limitatif de la présente invention.

### EXEMPLE 1

On prépare une composition cosmétique selon l'invention à partir des ingrédients indiqués ci-dessous dont les quantités sont indiquées en pour cent en poids, par rapport au poids total de la composition.

| Composition | Quantité de matières |
|---|---|
| Dihexadecanoate de [(5-amino-6- méthoxy -6-oxohexyl)imino]bis-2-hydroxypropane-3,1-diyl | 3% en M.A |
| Eau | qsp 100 |

On immerge dans 15g de la composition une mèche de cheveux décolorés (SA20) de 0,5g, préalablement mouillés. Le traitement est réalisé à 30°C pendant 15 minutes. La mèche est ensuite rincée et peignée. On observe que la mèche humide présente un toucher lisse, elle est souple et facile à démêler alors que le témoin dans l'eau seule est rugueux et difficile à démêler.

### EXEMPLE 2

On prépare une composition cosmétique selon l'invention à partir des ingrédients indiqués ci-dessous dont les quantités sont indiquées en pour cent en poids, par rapport au poids total de la composition.

| Composition | Quantité de matières |
|---|---|
| Mélange composé de 6% d'hexadécanoate de 3-[(5-amino-6-methoxy-6-oxohexyl)(2,3-dihydroxypropyl)amino]-2-hydroxypropyl, de 55% de dihexadecanoate de [(5-amino-6-méthoxy -6-oxohexyl)imino]bis-2-hydroxypropane-3,1-diyl et de 36% d'un mélange de palmitate de 3-{(5-amino-6-méthoxy-6-oxohexyl)[2,3-bis(palmitoyloxy)propyl]amino}-2-hydroxypropyl et de tétrahexadécanoate de [(5-amino-6-méthoxy-6-oxohexyl)imino]dipropane-3,1,2-triyl | 3% en M.A |
| Eau | qsp 100 |

On immerge dans 15g de la composition une mèche de cheveux décolorés (SA20) de 0,5g, préalablement mouillés. Le traitement est réalisé à 30°C pendant 15 minutes. La mèche est ensuite rincée et peignée. On observe que la mèche humide présente un toucher lisse, elle est souple et facile à démêler alors que le témoin dans l'eau seule est rugueux et difficile à démêler.

### EXEMPLE 3

On prépare une composition cosmétique selon l'invention à partir des ingrédients indiqués ci-dessous dont les quantités sont indiquées en pour cent en poids, par rapport au poids total de la composition.

| Composition | Quantité de matières |
|---|---|
| Mélange composé de 0,7% d'hexadécanoate de 3-[(5-amino-6-methoxy-6-oxohexyl)(2,3-dihydroxypropyl)amino]-2-hydroxypropyl, de 26% de dihexadecanoate de [(5-amino-6-méthoxy -6-oxohexyl)imino]bis-2-hydroxypropane-3,1-diyl et de 70% d'un mélange de palmitate de 3-{(5-amino-6-méthoxy-6-oxohexyl)[2,3-bis(palmitoyloxy)propyl]amino}-2-hydroxypropyl et de tétrahexadécanoate de [(5-amino-6-méthoxy-6-oxohexyl)imino]dipropane-3,1,2-triyl | 3% en M.A |
| Eau | qsp 100 |

On immerge dans 15g de la composition une mèche de cheveux décolorés (SA20) de 0,5g, préalablement mouillés. Le traitement est réalisé à 30°C pendant 15 minutes. La mèche est ensuite rincée et peignée. On observe que la mèche humide présente un toucher lisse, elle est souple et facile à démêler alors que le témoin dans l'eau seule est rugueux et difficile à démêler.

## Revendications

1. Utilisation pour le conditionnement des fibres kératiniques, en particulier des fibres kératiniques humaines telles que les cheveux d'un ou plusieurs composés dérivés de lysine de formule (I) suivante : 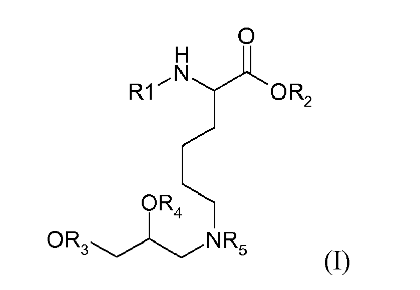 dans laquelle :
R₁ représente un atome d'hydrogène ou un groupement protecteur ;
R₂ représente un atome d'hydrogène ; un radical alkyle en C₁-C₆ linéaire ou un radical alkyle en C₃-C₆ ramifié ;
R₃ et R₄ représentent, indépendamment l'un de l'autre, un atome d'hydrogène ; un groupement acyle en C₂-C₂₅ linéaire, saturé ou insaturé, ou un groupement acyle en C₄-C₂₅ ramifié, saturé ou insaturé ;
R₅ représente un atome d'hydrogène ou un radical CH₂CH(OR₆)CH₂OR₇ ;
R₆ et R₇ représentent, indépendamment l'un de l'autre, un atome d'hydrogène ; un groupement acyle en C₃-C₂₅ linéaire, saturé ou insaturé, ou un groupement acyle en C₄-C₂₅ ramifié, saturé ou insaturé ;
R₁, R₂, R₃, R₄, R₅ et éventuellement R₆ et R₇ ne peuvent pas représenter simultanément un atome d'hydrogène ;
ainsi que leurs sels et/ou leurs solvates.

2. Utilisation selon la revendication 1, **caractérisée en ce que** le groupement protecteur est choisi parmi un groupement acyle en C₂-C₆, linéaire ou ramifié, de préférence un groupement acétyle ; un groupement alkyle en C₁-C₆, linéaire ou ramifié, de préférence un groupement méthyle ou éthyle ; un groupement carboxyalcoxy en C₁-C₆ ; un groupement alcoxy(C₁-C₆)carbonyle ; un groupement alkyle en C₁-C₆, linéaire ou ramifié, interrompu par un ou plusieurs atomes d'azote et/ou un groupement carboxy ; un groupement sulfonyle en C₂-C₆ ; un groupement aryle.

3. Utilisation selon la revendication 1, **caractérisée en ce que** R₁ représente un atome d'hydrogène ; R₂ représente un radical alkyle en C₁-C₄ linéaire, de préférence un groupement méthyle ou éthyle, ou un radical alkyle en C₃-C₄ ramifié, de préférence un groupement tertiobutyle ; R₃ et R₄ représentent, indépendamment l'un de l'autre, un atome d'hydrogène ou un groupement acyle en C₁₂-C₂₂, de préférence en C₁₆-C₂₂, linéaire ou ramifié, saturé ou insaturé ; R₅ représente un atome d'hydrogène ou groupement CH₂CH(OR₆)CH₂OR₇ ; R₆ et R₇ représentent, indépendamment l'un de l'autre, un atome d'hydrogène ou un groupement acyle en C₁₂-C₂₂, de préférence en C₁₆-C₂₂, linéaire ou ramifié, saturé ou insaturé ; R₁, R₂, R₃, R₄, R₅ et éventuellement R₆ et R₇ ne peuvent pas représenter simultanément un atome d'hydrogène.

4. Utilisation selon l'une quelconque des revendications précédentes, **caractérisée en ce que** les composés de formule (I) sont choisis dans le groupe formé par :
- l'hexadecanoate de 3-[(5-amino-6-tert-butoxy-6-oxohexyl)(2,3-dihydroxypropyl)amino]-2-hydroxypropyl ;
- le dodécanoate de 3-[(5-amino-6-tert-butoxy-6-oxohexyl)(2,3-dihydroxypropyl)amino]-2-hydroxypropyl ;
- l'hexadécanoate de 3-[(5-amino-6-methoxy-6-oxohexyl)(2,3-dihydroxypropyl)amino]-2-hydroxypropyl ;
- le dodécanoate de 3-[(5-amino-6-methoxy-6-oxohexyl)(2,3-dihydroxypropyl)amino]-2-hydroxypropyl ;
- l'hexadécanoate de 3-[(5-amino-6-ethoxy-6-oxohexyl)(2,3-dihydroxypropyl)amino]-2-hydroxypropyl ;
- le dodécanoate de 3-[(5-amino-6-ethoxy-6-oxohexyl)(2,3-dihydroxypropyl)amino]-2-hydroxypropyl ;
- le dihexadécanoate de [(5-amino-6-tert-butoxy-6-oxohexyl)imino]bis-2-hydroxypropane-3,1-diyl ;
- le didodécanoate de [(5-amino-6-tert-butoxy-6-oxohexyl)imino]bis-2-hydroxypropane 3,1-diyl ;
- le dihexadécanoate de [(5-amino-6-méthoxy-6-oxohexyl) imino]bis-2-hydroxypropane-3,1-diyl
- le didodécanoate de [(5-amino-6-méthoxy-6-oxohexyl) imino]bis-2-hydroxypropane-3,1-diyl ;
- le dihexadécanoate de [(5-amino-6-éthoxy-6-oxohexyl) imino]bis-2-hydroxypropane-3,1-diyl ;
- le didodécanoate de [(5-amino-6-éthoxy-6-oxohexyl) imino]bis-2-hydroxypropane-3,1-diyl ;
- le palmitate de 3-{(5-amino-6-tert-butoxy-6-oxohexyl)[2,3-bis(palmitoyloxy)propyl]amino}-2-hydroxypropyl ;
- le laurate de 3-{(5-amino-6-tert-butoxy-6-oxohexyl)[2,3-bis(dodecanoyloxy)propyl]amino}-2-hydroxypropyl ;
- le palmitate de 3-{(5-amino-6-méthoxy-6-oxohexyl)[2,3-bis(palmitoyloxy)propyl]amino}-2-hydroxypropyl ;
- le laurate de 3-{(5-amino-6-méthoxy-6-oxohexyl)[2,3-bis(dodecanoyloxy)propyl]amino}-2-hydroxypropyl ;
- le palmitate de 3-{(5-amino-6-éthoxy-6-oxohexyl)[2,3-bis(palmitoyloxy)propyl]amino}-2-hydroxypropyl ;
- le laurate de 3-{(5-amino-6-éthoxy-6-oxohexyl)[2,3-bis(dodecanoyloxy)propyl]amino}-2-hydroxypropyl ;
- le tétrahexadécanoate de [(5-amino-6-tert-butoxy-6-oxohexyl)imino]dipropane-3,1,2-triyl ;
- le tétradodécanoate de [(5-amino-6-tert-butoxy-6-oxohexyl)imino]dipropane-3,1,2-triyl ;
- le tétrahexadécanoate de [(5-amino-6-méthoxy-6-oxohexyl)imino]dipropane-3,1,2-triyl ;
- le tétradodécanoate de [(5-amino-6-méthoxy-6-oxohexyl)imino]dipropane-3,1,2-triyl ;
- le tétrahexadécanoate de [(5-amino-6-éthoxy-6-oxohexyl)imino]dipropane-3,1,2-triyl ;
- le tétradodecanoate de [(5-amino-6-éthoxy-6-oxohexyl)imino]dipropane-3,1,2-triyl ;
- le palmitate de 3-[(5-amino-6-tert-butoxy-6-oxohexyl)amino]-2-hydroxypropyl ;
- le laurate de 3-[(5-amino-6-tert-butoxy-6-oxohexyl)amino]-2-hydroxypropyl ;
- le palmitate de 3-[(5-amino-6-méthoxy-6-oxohexyl)amino]-2-hydroxypropyl ;
- le laurate de 3-[(5-amino-6-méthoxy-6-oxohexyl)amino]-2-hydroxypropyl ;
- le palmitate de 3-[(5-amino-6-éthoxy-6-oxohexyl)amino]-2-hydroxypropyl ;
- le laurate de 3-[(5-amino-6-éthoxy-6-oxohexyl)amino]-2-hydroxypropyl ;
- le dihexadécanoate de 3-[(5-amino-6-tert-butoxy-6-oxohexyl)amino]propane-1,2-diyl ;
- le didodécanoate de 3-[(5-amino-6-tert-butoxy-6-oxohexyl)amino]propane-1,2-diyl ;
- le dihexadécanoate 3-[(5-amino-6-méthoxy-6-oxohexyl)amino] propane-1,2-diyl ;
- le didodécanoate de 3-[(5-amino-6-méthoxy-6-oxohexyl) amino]propane-1,2-diyl ;
- le dihexadécanoate 3-[(5-amino-6-éthoxy-6-oxohexyl) amino]propane-1,2-diyl ;
- le didodécanoate de 3-[(5-amino-6-éthoxy-6-oxohexyl) amino]propane-1,2-diyl
- le docosanoate de 3-[(5-amino-6-tert-butoxy-6-oxohexyl)(2,3-dihydroxypropyl)amino]-2-hydroxypropyl ;
- le docosanoate de 3-[(5-amino-6-methoxy-6-oxohexyl)(2,3-dihydroxypropyl)amino]-2-hydroxypropyl ;
- le docosanoate de 3-[(5-amino-6-ethoxy-6-oxohexyl)(2,3-dihydroxypropyl)amino]-2-hydroxypropyl ;
- le didocosanoate de [(5-amino-6-tert-butoxy-6-oxohexyl)imino]bis-2-hydroxypropane-3,1-diyl ;
- le docosanoate de 3-{(5-amino-6-tert-butoxy-6-oxohexyl)[2,3-bis(docosanoyloxy)propyl]amino}-2-hydroxypropyl ;
- le didocosanoate de [(5-amino-6-méthoxy-6-oxohexyl) imino]bis-2-hydroxypropane-3,1-diyl ;
- le docosonoate de 3-{(5-amino-6-méthoxy-6-oxohexyl)[2,3-bis(docosanoyl)oxy)propyl]amino}-2-hydroxypropyl ;
- le didocosanoate de [(5-amino-6-éthoxy-6-oxohexyl) imino]bis-2-hydroxypropane-3,1-diyl ;
- le docosonoate de 3-{(5-amino-6-éthoxy-6-oxohexyl)[2,3-bis(docosanoyl)oxy)propyl]amino}-2-hydroxypropyl ;
- le docosanoate de 3-[(5-amino-6-méthoxy-6-oxohexyl) amino]-2-hydroxypropyl ;
- le didocosanoate de 3-[(5-amino-6-tert-butoxy-6-oxohexyl)amino]propane-1,2-diyl ;
- le tétradocosanoate de [(5-amino-6-tert-butoxy-6-oxohexyl)imino]dipropane-3,1,2-triyl ;
- le docosanoate de 3-[(5-amino-6-tert-butoxy-6-oxohexyl)amino]-2-hydroxypropyl ;
- le didocosanoate de 3-[(5-amino-6- méthoxy -6-oxohexyl) amino]propane-1,2-diyl
- le tétradocosanoate de [(5-amino-6-méthoxy-6-oxohexyl)imino]dipropane-3,1,2-triyl ;
- le docosanoate de 3-[(5-amino-6-éthoxy-6-oxohexyl)amino]-2-hydroxypropyl ;
- le didocosanoate de 3-[(5-amino-6-éthoxy-6-oxohexyl)amino]propane-1,2-diyl ;
- le tétradocosanoate de [(5-amino-6-éthyl-6-oxohexyl)imino]dipropane-3,1,2-triyl.

5. Utilisation selon l'une quelconque des revendications précédentes, **caractérisée en ce que** les sels sont des sels inorganiques choisis parmi les halogénohydrates, les carbonates, les hydrogénocarbonates, les sulfates, les hydrogénophosphates et les phosphates.

6. Utilisation selon l'une quelconque des revendications 1 à 4, **caractérisée en ce que** les sels sont des sels organiques choisis parmi les sels d'acides organiques tels que les citrates, les lactates, les glycolates, les gluconates, les acétates, les propionates, les fumarates, les oxalates et les tartrates.

7. Utilisation selon l'une quelconque des revendications 1 à 4, **caractérisée en ce que** les solvates sont choisis parmi les hydrates, les alcoolates et les hydroalcoolates.

8. Composition cosmétique pour le conditionnement des fibres kératiniques, en particulier des fibres kératiniques humaines telles que les cheveux, comprenant, dans un milieu cosmétiquement acceptable, un ou plusieurs composés tels que définis selon l'une quelconque des revendications précédentes et un ou plusieurs adjuvants cosmétiques.

9. Composition cosmétique selon la revendication 8, **caractérisée en ce que** le ou les adjuvants cosmétiques est ou sont choisis parmi les tensioactifs anioniques, cationiques autres que les composés de formule (I), amphotères, non-ioniques, les polymères cationiques, anioniques, amphotères ou zwittérioniques, non ioniques et les silicones.

10. Procédé de conditionnement des fibres kératiniques, en particulier des fibres kératiniques humaines telles que les cheveux, consistant à appliquer sur les fibres sèches ou humides, une composition cosmétique comprenant un ou plusieurs composés tels que définis selon l'une quelconque des revendications 1 à 5, à rincer ou non après un éventuel temps de pose ou après un éventuel séchage.

11. Utilisation de la composition telle que définie selon la revendication 8 ou 9 pour le conditionnement des fibres kératiniques.

12. Composé dérivé de lysine **caractérisé en ce qu'**il est choisi parmi :
- l'hexadecanoate de 3-[(5-amino-6-tert-butoxy-6-oxohexyl)(2,3-dihydroxypropyl)amino]-2-hydroxypropyl ;
- le dodécanoate de 3-[(5-amino-6-tert-butoxy-6-oxohexyl)(2,3-dihydroxypropyl)amino]-2-hydroxypropyl ;
- l'hexadécanoate de 3-[(5-amino-6-methoxy-6-oxohexyl)(2,3-dihydroxypropyl)amino]-2-hydroxypropyl ;
- le dodécanoate de 3-[(5-amino-6-methoxy-6-oxohexyl)(2,3-dihydroxypropyl)amino]-2-hydroxypropyl ;
- l'hexadécanoate de 3-[(5-amino-6-ethoxy-6-oxohexyl)(2,3-dihydroxypropyl)amino]-2-hydroxypropyl ;
- le dodécanoate de 3-[(5-amino-6-ethoxy-6-oxohexyl)(2,3-dihydroxypropyl)amino]-2-hydroxypropyl ;
- le dihexadécanoate de [(5-amino-6-tert-butoxy-6-oxohexyl)imino]bis-2-hydroxypropane-3,1-diyl ;
- le didodécanoate de [(5-amino-6-tert-butoxy-6-oxohexyl)imino]bis-2-hydroxypropane 3,1-diyl ;
- le dihexadécanoate de [(5-amino-6-méthoxy-6-oxohexyl) imino]bis-2-hydroxypropane-3,1-diyl
- le dihexadécanoate de [(5-amino-6-éthoxy-6-oxohexyl) imino]bis-2-hydroxypropane-3,1-diyl ;
- le didodécanoate de [(5-amino-6-éthoxy-6-oxohexyl) imino]bis-2-hydroxypropane-3,1-diyl ;
- le palmitate de 3- {(5-amino-6-tert-butoxy-6-oxohexyl)[2,3-bis(palmitoyloxy)propyl]amino}-2-hydroxypropyl ;
- le laurate de 3-{(5-amino-6-tert-butoxy-6-oxohexyl)[2,3-bis(dodecanoyloxy) propyl]amino}-2-hydroxypropyl ;
- le palmitate de 3-{(5-amino-6-méthoxy-6-oxohexyl)[2,3-bis(palmitoyloxy)propyl]amino}-2-hydroxypropyl ;
- le laurate de 3-{(5-amino-6-méthoxy-6-oxohexyl)[2,3-bis(dodecanoyloxy)propyl]amino}-2-hydroxypropyl ;
- le palmitate de 3-{(5-amino-6-éthoxy-6-oxohexyl)[2,3-bis(palmitoyloxy)propyl]amino}-2-hydroxypropyl ;
- le laurate de 3-{(5-amino-6-éthoxy-6-oxohexyl)[2,3-bis(dodecanoyloxy)propyl]amino}-2-hydroxypropyl ;
- le tétrahexadécanoate de [(5-amino-6-tert-butoxy-6-oxohexyl)imino]dipropane-3,1,2-triyl ;
- le tétradodécanoate de [(5-amino-6-tert-butoxy-6-oxohexyl)imino]dipropane-3,1,2-triyl ;
- le tétrahexadécanoate de [(5-amino-6-méthoxy-6-oxohexyl)imino]dipropane-3,1,2-triyl ;
- le tétradodécanoate de [(5-amino-6-méthoxy-6-oxohexyl)imino]dipropane-3,1,2-triyl ;
- le tétrahexadécanoate de [(5-amino-6-éthoxy-6-oxohexyl)imino]dipropane-3,1,2-triyl ;
- le tétradodecanoate de [(5-amino-6-éthoxy-6-oxohexyl)imino]dipropane-3,1,2-triyl ;
- le palmitate de 3-[(5-amino-6-tert-butoxy-6-oxohexyl)amino]-2-hydroxypropyl ;
- le laurate de 3-[(5-amino-6-tert-butoxy-6-oxohexyl)amino]-2-hydroxypropyl ;
- le palmitate de 3-[(5-amino-6-méthoxy-6-oxohexyl)amino]-2-hydroxypropyl ;
- le laurate de 3-[(5-amino-6-méthoxy-6-oxohexyl)amino]-2-hydroxypropyl ;
- le palmitate de 3-[(5-amino-6-éthoxy-6-oxohexyl)amino]-2-hydroxypropyl ;
- le laurate de 3-[(5-amino-6-éthoxy-6-oxohexyl)amino]-2-hydroxypropyl ;
- le dihexadécanoate de 3-[(5-amino-6-tert-butoxy-6-oxohexyl)amino]propane-1,2-diyl ;
- le didodécanoate de 3-[(5-amino-6-tert-butoxy-6-oxohexyl)amino]propane-1,2-diyl ;
- le dihexadécanoate 3-[(5-amino-6-méthoxy-6-oxohexyl)amino] propane-1,2-diyl ;
- le didodécanoate de 3-[(5-amino-6-méthoxy-6-oxohexyl) amino]propane-1,2-diyl ;
- le dihexadécanoate 3-[(5-amino-6-éthoxy-6-oxohexyl) amino]propane-1,2-diyl ;
- le didodécanoate de 3-[(5-amino-6-éthoxy-6-oxohexyl) amino]propane-1,2-diyl
- le docosanoate de 3-[(5-amino-6-tert-butoxy-6-oxohexyl)(2,3-dihydroxypropyl)amino]-2-hydroxypropyl ;
- le docosanoate de 3-[(5-amino-6-methoxy-6-oxohexyl)(2,3-dihydroxypropyl)amino]-2-hydroxypropyl ;
- le docosanoate de 3-[(5-amino-6-ethoxy-6-oxohexyl)(2,3-dihydroxypropyl)amino]-2-hydroxypropyl ;
- le didocosanoate de [(5-amino-6-tert-butoxy-6-oxohexyl)imino]bis-2-hydroxypropane-3,1-diyl ;
- le docosanoate de 3-{(5-amino-6-tert-butoxy-6-oxohexyl)[2,3-bis(docosanoyloxy)propyl]amino}-2-hydroxypropyl ;
- le didocosanoate de [(5-amino-6-méthoxy-6-oxohexyl) imino]bis-2-hydroxypropane-3,1-diyl ;
- le docosonoate de 3-{(5-amino-6-méthoxy-6-oxohexyl)[2,3-bis(docosanoyl)oxy)propyl]amino}-2-hydroxypropyl ;
- le didocosanoate de [(5-amino-6-éthoxy-6-oxohexyl) imino]bis-2-hydroxypropane-3,1-diyl ;
- le docosonoate de 3-{(5-amino-6-éthoxy-6-oxohexyl)[2,3-bis(docosanoyl)oxy)propyl]amino}-2-hydroxypropyl ;
- le docosanoate de 3-[(5-amino-6-méthoxy-6-oxohexyl) amino]-2-hydroxypropyl ;
- le didocosanoate de 3-[(5-amino-6-tert-butoxy-6-oxohexyl)amino]propane-1,2-diyl ;
- le tétradocosanoate de [(5-amino-6-tert-butoxy-6-oxohexyl)imino]dipropane-3,1,2-triyl ;
- le docosanoate de 3-[(5-amino-6-tert-butoxy-6-oxohexyl)amino]-2-hydroxypropyl ;
- le didocosanoate de 3-[(5-amino-6- méthoxy -6-oxohexyl) amino]propane-1,2-diyl
- le tétradocosanoate de [(5-amino-6-méthoxy-6-oxohexyl)imino]dipropane-3,1,2-triyl ;
- le docosanoate de 3-[(5-amino-6-éthoxy-6-oxohexyl)amino]-2-hydroxypropyl ;
- le didocosanoate de 3-[(5-amino-6-éthoxy-6-oxohexyl)amino]propane-1,2-diyl ;
- le tétradocosanoate de [(5-amino-6-éthyl-6-oxohexyl)imino]dipropane-3,1,2-triyl ;
ou un mélanges de ces composés.

13. Mélange de composés dérivés de lysine de formule (I) comprenant de 0,5 à 15% en poids de mono-esters, de 25 à 99,5% en poids de di-esters, de 0,5 à 70% en poids de triesters et de 0,5 à 70% en poids de tétra-esters, par rapport au poids total du mélange.

14. Mélange selon la revendication 13, **caractérisé en ce qu'**il comprend 6 % en poids d'hexadécanoate de 3-[(5-amino-6-methoxy-6-oxohexyl)(2,3-dihydroxypropyl)amino]-2-hydroxypropyl, 55% en poids de dihexadecanoate de [(5-amino-6- méthoxy-6-oxohexyl)imino]bis-2-hydroxypropane-3,1-diyl et 36% en poids d'un mélange de palmitate de 3-{(5-amino-6-méthoxy-6-oxohexyl)[2,3-bis(palmitoyloxy)propyl]amino}-2-hydroxypropyl et de tétrahexadécanoate de [(5-amino-6-méthoxy-6-oxohexyl)imino]dipropane-3,1,2-triyl.

15. Utilisation en cosmétique d'un ou plusieurs composés dérivés de lysine tels que définis selon les revendications 12 à 14.
